# EUROPEAN PATENT SPECIFICATION

(11) **EP 3 496 606 B1**
(45) Date of publication and mention of the grant of the patent: **09.12.2026**
(21) Application number: 17755606.5
(22) Date of filing: 10.08.2017
(51) Int. Cl.: A61B 5/00, A61B 5/021, A61B 5/024, A61B 5/107, A61B 8/00, A61B 8/06, A61B 8/08, A61B 8/12, G16H 50/30

(54) **SYSTEM FOR PATIENT FLUID MANAGEMENT**
SYSTEM ZUM PATIENTENFLUIDMANAGEMENT
SYSTÈME DE GESTION DES FLUIDES CHEZ UN PATIENT

(30) Priority: 11.08.2016 US 201662373436 P; 29.11.2016 US 201662427631 P; 19.07.2017 US 201762534329 P
(43) Date of publication of application: 19.06.2019
(73) Proprietor: Foundry Innovation & Research 1, Ltd., Dublin 2 (IE)
(72) Inventor: DEEM, Mark, E., Menlo Park, CA 94025 (US); HANLEY, Conor, M., Dublin 2 (IE); SWEENEY, Fiachra, M., Dublin 2 (IE); GRAINGER, Jeffry, J., Menlo Park, CA 94025 (US); BRITTON, John, R., Dublin 2 (IE); KENT, Annette, Dublin 18 (IE); GIFFORD, Hanson, S., III, Menlo Park, CA 94025 (US)
(74) Representative: Tomkins & Co
(86) International application number: PCT/US2017/046204
(87) International publication number: WO 2018/031714

(56) References cited:
- WO-A1-2016/131020
- US-A1- 2011 306 867
- US-A1- 2013 303 915
- J. M. BRENNAN: "Handcarried Ultrasound Measurement of the Inferior Vena Cava for Assessment of Intravascular Volume Status in the Outpatient Hemodialysis Clinic", CLINICAL JOURNAL OF THE AMERICAN SOCIETY OF NEPHROLOGY, vol. 1, no. 4, 21 June 2006 (2006-06-21), pages 749 - 753, XP055193327, ISSN: 1555-9041, DOI: 10.2215/CJN.00310106

## Description

### FIELD OF THE DISCLOSURE

Embodiments disclosed herein relate to systems and methods for patient fluid management, for example in connection with heart failure or renal failure conditions, as well as other disease states requiring careful management of patient fluid balance.

### BACKGROUND

A significant challenge in the treatment of acute heart failure patients is the management of the patient fluid volume. Similar challenges are also presented in the treatment of renal failure patients, and, in fact, studies have shown a direct correlation, and potentially causal relationship, between heart and renal failure conditions with respect to patient fluid management [e.g., Silverberg, et al., The association between congestive heart failure and chronic renal disease, Curr. Opin. Nephrol. Hypertens. (2004) 13:163-170]. Acute heart or renal failure can lead to peripheral and pulmonary edema if not properly treated, but too aggressive of a treatment can lead to a hypovolemic state in which the patient lacks sufficient fluid volume. Treatments may include dialysis, ultrafiltration, diuretics and other drug administration. For longer term patients, fluid and dietary intake also may be monitored and modulated. Traditionally, diagnostic techniques used in monitoring fluid status were based on various externally observable symptoms (e.g., jugular vein distention, edema, patient weight change). Also, central venous catheterization (CVC) to monitor central venous pressure (CVP) has been used as a fluid status indicator. However, there are a number of serious risks associated with CVC, such as infection and thrombosis, and reliance on externally observable or measurable symptoms presents an obvious drawback in that the observable response to a therapy is often significantly delayed relative to acute changes in physiological status.

Monitoring fluid status can also be used as a predictor for onset of acute decompensated heart failure (ADHF), which is a significant factor driving rehospitalization of heart failure patients. There is potential to significantly reduce hospitalizations if there is a sufficiently early signal of increasing patient fluid volume. However, drawbacks of traditional diagnostic tools as mentioned above make such tools relatively ineffective as early predictors of ADHF.

In an attempt to overcome risks and drawbacks associated with more traditional diagnostic techniques, different types of diagnostic devices or techniques have been developed to measure central venous pressure (CVP) [e.g., Shuros, et al., Coronary Vein Hemodynamic Sensor, US 20090/01497666, Jun. 11, 2009] or pulmonary artery pressure (PAP) *[e.g.,* Abraham, et al., Wireless pulmonary artery haemodynamic monitoring in chronic heart failure: a randomized controlled trial, Lancet (2011) 377:658-66]. Also, research using external ultrasound observation of the Inferior Vena Cava (IVC) has led to a general understanding of a correlation between the IVC volume and patient health in terms of proper fluid balance *[e.g.,* Feissel et al., The respiratory variation in inferior vena cava diameter as a guide to fluid therapy, Intensive Care Med (2004) 30:1834-1837]. Based on this understanding, external ultrasound systems are now sometimes used in emergency treatment situations to provide the attending physicians with information on patient fluid state. In addition, more recent techniques have been proposed in which devices indirectly measure vessel pressure or volume based on changes in impedance in response to an applied current within the vessel.

While devices and techniques now available may offer advantages over more traditional techniques based on observation of externally visible symptoms, each has its own disadvantages that limit effectiveness as a diagnostic tool to support more aggressive therapies. Many newer monitoring devices or techniques either do not provide sufficiently accurate data to allow early warning of changes in patient stability or do not provide guidance with respect to a particular type of intervention *[see, e.g.* , Marik, et al., Does Central Venous Pressure Predict Fluid Responsiveness?*: A Systematic Review of the Literature and the Tale of Seven Mares, Chest (2008) 134(1):172-178]. Examples include that impedance-based devices have not shown sufficient sensitivity and PAP measurements do not provide a warning of hypovolemia. External measurement of IVC dimensions with external ultrasound systems is heavily reliant on proper and consistent positioning of the patient and the imaging device, both initially and over the period of monitoring, and may not always provide accurate prediction of fluid state *[e.g.,*Blehar, et al, Inferior vena cava displacement during respirophasic ultrasound imaging, Critical Ultrasound Journal (2012) 4:18]. It is also impractical for use as a longer term diagnostic tool for regular (e.g. daily) monitoring of patients who are not hospitalized. An other device sensing the physical dimensions of a blood vessel is disclosed in US 2011/0306867 A1.

### SUMMARY OF THE DISCLOSURE

Embodiments disclosed herein include a system for managing patient body fluid volume, comprising a wireless sensing device, a sensing control module and a patient management system. The wireless sensing device is configured to be positioned within a patient's IVC to measure a physical dimension of the IVC and generate a measurement signal indicative of the measured IVC physical dimension. The sensing device control module is configured to wirelessly communicate with the sensing device to at least receive the measurement signal from the sensing device. The patient management system may include a processor and memory, and is configured to receive or generate measurement data representative of the measured IVC physical dimension based on the measurement signal, to receive patient specific information, and to execute instructions contained in the memory responsive to received patient specific information and the measurement data. The instructions contained in memory may include instructions to prompt for initiation of a sensor measurement of the IVC physical dimension and generate an alert signal when the measured IVC physical dimension is outside predetermined clinical limits for the patient. The instructions may further include instructions to wirelessly communicate with the implanted sensing device to monitor changes in the measured IVC dimension throughout an euvolemic region defined for said patient. In some embodiments, the physical dimension of the patient's IVC is at least one of IVC diameter or IVC area.

In another, alternative embodiment, a system for managing patient body fluid volume includes a processor and memory configured to communicate with the implanted wireless sensing device and the sensing device control module. The processor may be configured to receive measurement data representing the IVC physical dimension measurement by the sensing device and execute instructions contained in the memory responsive that measurement data. The processor also may be configured to receive patient specific information. The instructions stored in memory may comprise defining euvolemic, hypovolemic and hypervolemic regions for the patient wherein the euvolemic range, the hypovolemic range and the range are correlated to IVC diameter or volume measurements for the patient, identifying a hypovolemic warning zone for the patient encompassing a portion of the defined euvolemic region at a lower end of the euvolemic region adjacent the hypovolemic region, identifying a hypervolemic warning zone for the patient encompassing a portion of the defined euvolemic region at an upper end of the euvolemic region adjacent the hypervolemic region, generating an alert signal when measured IVC diameter or volume falls within either of the hypovolemic warning zone or hypervolemic warning zone of the defined euvolemic region before the patient fluid state reaches one of the hypovolemic region or hypervolemic region, respectively.

In some embodiments the wireless sensing device may comprise an ultrasound transducer and anchor element configured to anchor the ultrasound transducer in the IVC in a fixed position relative to the IVC wall. In other embodiments, the wireless sensing device may comprise a resilient coil forming a resonant circuit, wherein the resilient coil is configured to engage the wall of the IVC and deform therewith to provide a variable characteristic frequency correlated to an IVC physical dimension in response to activation of the resonant circuit. Such a system may further include an antenna configured to activate the resonant circuit and receive the signal representative of the measured IVC physical dimension in response to activation.

Further alternative embodiments disclosed herein include methods of managing patient body fluid volume. One embodiment of the disclosed methods may include directly measuring a physical dimension of the patient's IVC with a sensing device implanted within the IVC, wirelessly communicating with the implanted sensing device to monitor changes in the measured IVC dimension throughout an euvolemic region defined for said patient, and generating an alert signal when measured IVC diameter or area approaches or falls outside predetermined clinical limits for the patient. The alert signal may be direct to a healthcare provider or to the patient. When directed to the patient, the alert signal may include a prompt to initiate a patient self-directed treatment.

In some embodiments the predetermined clinical limits may include at least a first, lower limit indicative of the patient fluid state trending towards hypovolemia, and a second, higher limit indicative of the patient fluid state trending towards hypervolemia. Further, each of the predetermined limits falls within the euvolemic range defined for the patient. In such embodiments, further method steps may comprise setting (1) a hypovolemic warning zone for the patient encompassing a portion of the defined euvolemic region at the lower end of the euvolemic region adjacent a hypovolemic region and (2) a hypervolemic warning zone for the patient encompassing a portion of the defined euvolemic region at the upper end of the euvolemic region adjacent a hypervolemic region, wherein the euvolemic range, hypovolemic range and hypervolemic range are correlated to IVC physical dimension measurements for the patient.

In another alternative embodiment disclosed herein, a diagnostic method, not part of the claimed invention, for monitoring patient body fluid volume comprises positioning a sensing device within the patient's IVC, wherein the sensing device is configured to measure at least one physical dimension of the IVC. Wirelessly monitoring changes in measured IVC physical dimension over time throughout an euvolemic region defined for said patient. Identifying (1) a hypovolemic warning zone for the patient encompassing a portion of the defined euvolemic region at the lower end of the euvolemic region adjacent a hypovolemic region and (2) a hypervolemic warning zone for the patient encompassing a portion of the defined euvolemic region at the upper end of the euvolemic region adjacent a hypervolemic region, wherein the euvolemic range, hypovolemic range and hypervolemic range are correlated to IVC physical dimension measurements for the patient. Another step in such a method as disclosed may include signaling a warning state when measured IVC physical dimension falls within either of the hypovolemic warning zone or hypervolemic warning zone of the defined euvolemic region before the patient fluid state reaches one of the hypovolemic region or hypervolemic region, respectively. The invention is defined by the appended claims.

### BRIEF DESCRIPTION OF THE DRAWINGS

For the purpose of illustrating the disclosed embodiments, the drawings show aspects thereof. It is to be understood, however, that the teachings of the present disclosure are not limited to the precise arrangements and instrumentalities shown in the drawings, wherein:
FIG. 1 is a schematic plot of patient fluid volume versus response for disclosed embodiments employing on IVC diameter or area measurement (curves A₁ and A₂) in comparison to prior pressure-based systems (curve B) and in general relationship to IVC collapsibility index (IVC CI, curve C).
FIG. 2 illustrates a hypothetical comparison of patient fluid volume over time in treatment for hypervolemia as between an IVC diameter or area measurement-based approach according to the present disclosure (curve X) and a typical pressure-based approach (curves Y and Z).
FIGS. 3A and 3B schematically illustrate alternative treatment embodiments employing titration of therapy based on disclosed systems.
FIG. 3C illustrates a treatment scenario based on disclosed system embodiments.
FIGS. 4A, 4B, 4C, 5A and 5B schematically illustrate embodiments of closed loop control of dialysis and therapy/treatment devices based on systems disclosed herein.
FIGS. 6A and 6B schematically depict components and possible arrangement of alternative system embodiments as disclosed herein.
FIG. 6C shows examples of screen shots from a patient's mobile device presenting patient prompts as part of a patient self-directed therapy algorithm.
FIG. 7 illustrates an exemplary algorithm for determination of IVC collapsibility (IVC CI) on which a treatment algorithm may be based.
FIG. 8 illustrates a possible treatment algorithm according to the present disclosure.
FIG. 9 illustrates an exemplary workflow utilizing a system employing an implanted IVC Volume Metric monitoring device as disclosed herein.
FIG. 10 schematically illustrates one embodiment of a local system for receiving signals/communicating with an implant according to embodiments disclosed herein.
FIG. 11 presents a block diagram of one embodiment of an IVC measurement implant.
FIGS. 12A, 12B, and 12C illustrate more details of further embodiments of IVC measurement implants according to the present disclosure.
FIG. 13 a block diagram of a computing system that can be used to implement any one or more of the methodologies disclosed herein and any one or more portions thereof.

### DESCRIPTION OF EMBODIMENTS

Embodiments disclosed herein include systems and methodologies allowing for regular, periodic or continuous monitoring of fluid volume more accurately than current techniques and with reduced lag time before changes in volume status are observed, thus providing earlier warning of hypervolemia or hypovolemia and enabling the modulation of patient treatments to permit more stable long term fluid management. Further, in acute situations, the methods and systems disclosed enable more rapid reduction of excessive intravascular volume and edema and restoration of more ideal fluid balance, with lessened risk of creating a hypovolemic state as can be created when patient "drying" treatments overshoot due to the response of patient monitoring devices or protocols.

A challenge presented by patients in decompensated heart failure is managing patient fluid balance, bringing down excess fluid volume as quickly as possible, but without overshooting and taking the patient into a potentially equally dangerous hypovolemic state. In the longer term management of heart failure, maintaining fluid balance is still a challenge, but in this case it involves maintaining the patient well within a safe fluid state without unintentionally migrating into a hypervolemic or hypovolemic state.

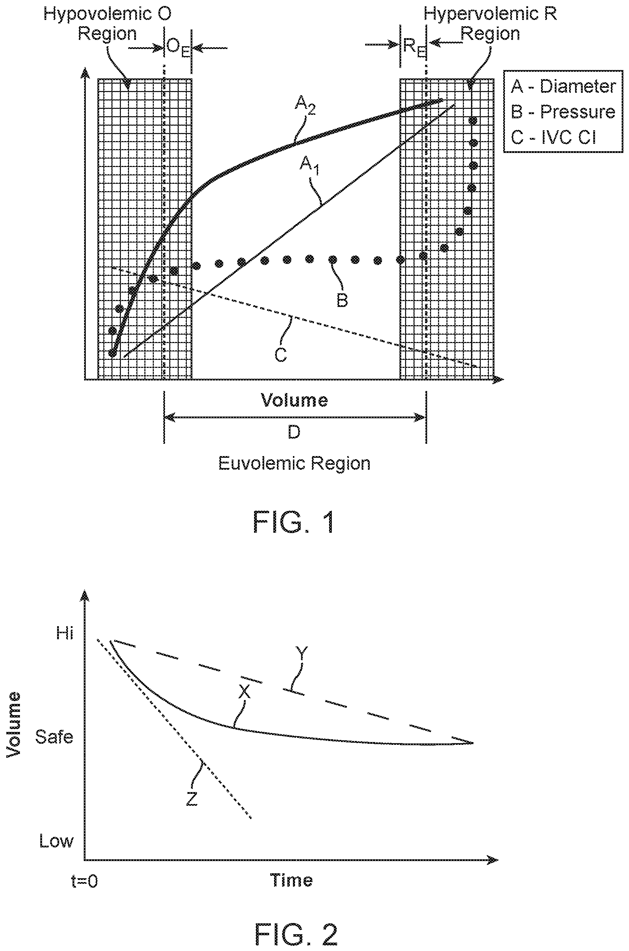

Existing clinical devices focused on pressure measurement present certain difficulties in meeting these challenges. Pressure measurements can be useful as an indicator of gross fluid volume change, and as a predictor of acute decompensation when fluid levels are already at a relatively high level. However, detectable changes in PAP can lag changes in physiological state to an extent that a patient may be in an early stage of the risk zone before the change is identified. Also, as shown in FIG. 1, the relationship between pressure and volume in the IVC is highly nonlinear over the entire range from hypovolemic to euvolemic to hypervolemic, exhibiting significant volume change within the mid-range, generally euvolemic state, with minimal corresponding change in pressure that can be measured. (See FIG. 1, curve B). For these reasons, PAP and CVP can be limited predictors of volume status and likelihood of acute decompensation before a near acute stage is reached.

While there is a general knowledge of a correlation between IVC diameter and patient health and fluid state, existing devices and techniques for monitoring patient fluid state across the full volume range have not led to treatment systems or methodologies permitting more rapid stabilization of acute patients and longer term maintenance without that avoids the critical fluid states and may thus lead to otherwise unnecessary treatments or hospitalizations.

"Euvolemia" refers to the normal fluid volume in a patient's body, and "euvolemic region" refers to a range of fluid volume within the patient that is clinically characterizable as normal or not requiring intervention. ("Euvolemia" is also sometimes referred to in the medical literature as "normovolemia.") The euvolemic region, as explained in more detail below, also a fluid state or volume range across which measurement of central venous pressure (CVP) in the IVC is generally non-responsive to changes in fluid volume. "Hypervolemia" refers to a state in which a patient's body fluid volume exceeds a normal range, and "hypervolemic range" refers to a range of fluid volume within the patient that is clinically characterizable as excessive. Intervention may be indicated when a patient trends towards, enters into or persists within the hypervolemic range. "Hypovolemia" refers to a state in which a patient's body fluid volume is below a normal range, and "hypovolemic range" refers to a range of fluid volume within the patient that is clinically characterizable as insufficient. As with the hypervolemic range, intervention also may be indicated when a patient trends towards, enters into or persists within the hypovolemic range. As is well-understood by clinicians and other persons of skill in the art, these body fluid states are not static nor are they uniform or in terms of absolute volume. While it is possible for a person of ordinary skill to assign approximate ranges of parameters generally corresponding to the different fluid states, it can be difficult in practice for ordinary skilled persons, with existing diagnostic tools and methods, to identify where a particular patient's fluid state may reside with respect to the euvolemic, hypovolemic and hypervolemic ranges..

In response to the need for more accurate devices with faster response times, the Assignee of the present disclosure has developed a number of new devices that provide fluid volume data based on direct measurement of physical dimensions of the IVC, such as the diameter or area. Examples of these new devices are described, for example, in PCT/US2016/017902, filed February 12, 2016, US Provisional Patent Application, Serial no. 62/427,631, filed November 29, 2016, and US Provisional Application, Serial No. 62/534,329, filed July 19, 2017 by the present Applicant. Devices of the types described in these prior disclosures facilitate new management and treatment techniques as described herein based on regular intermittent (e.g., daily) or substantially continuous (near real-time), direct feedback on IVC diameter.

In further alternative embodiments disclosed herein, patient fluid state can be further modulated based on a combination of IVC data with other monitoring signals; symptoms and clinical input, by use of IVC data as it is influenced by some stimulus (e.g., exercise; leg raises) to indicate either system capacitance or redistribution of fluid, by use of IVC measurements from an implanted sensor to transmit regular information locally to help the clinical management of patients, e.g. patients managing their own dialysis and/or diuresis at home, or by use of IVC measurement from an implanted sensor to control drug delivery *(e.g.,* like a closed loop implanted system for diabetes). Advantages achievable with disclosed systems and methods may include improved reduction of excessive intravascular blood volume in the clinical setting, through the controlled use of diuretics, more accurate management of blood volume in the home setting, through the monitoring of patients and use of a treatment algorithm, more rapid dialysis through the monitoring of volume and informed variation of dialysis rate.

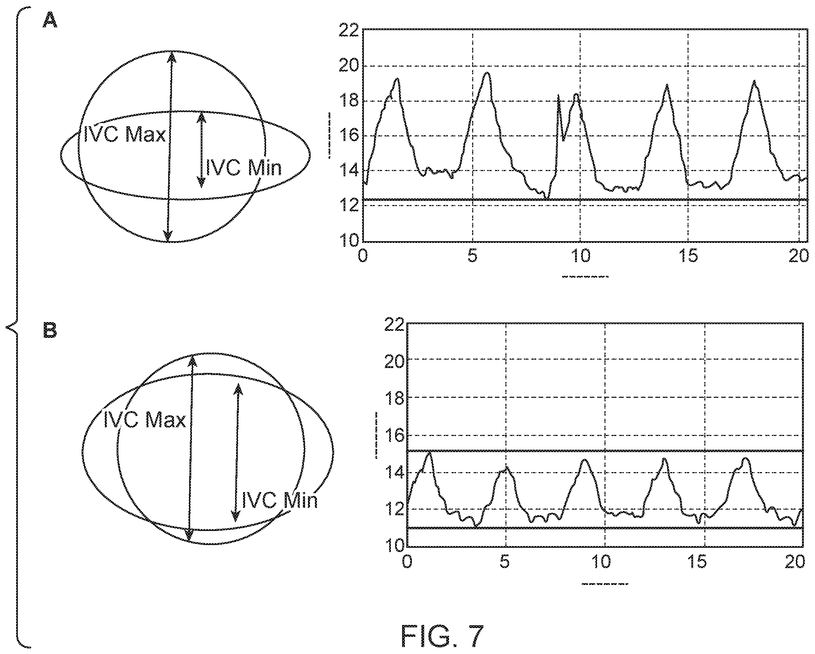

As an illustration of the presently disclosed methodology, FIG. 1 presents a schematic plot of patient fluid volume versus a number of responses. IVC diameter or measurement versus Volume are shown by curves A₁ and A₂ in comparison to IVC Pressure versus Volume (curve B) and the IVC collapsibility index versus Volume (IVC CI, curve C). The IVC collapsibility index (CI) is equal to the measured IVC dimension at maximum extension minus the same dimension at minimum extension divided by the maximum extension dimension. (CI= Max-Min/Max). Any single dimension measurement may be used, i.e. major diameter, minor diameter or area (see FIG. 7). It should be noted that FIG. 1 is intended only to summarize and illustrate overall relationships of the parameters discussed, and does not represent specific data points or data plotted to scale. (Curves A1 and A2 represent data from preclinical and benchtop testing conducted by the present Applicant based on prototype devices of a type described herein. Curve B is adapted from canine IVC results published by Moreno et al., Circ. Res. (1970) 27 (5): 1069-1080.

As can be seen in FIG. 1, the response of pressure-based diagnostic tools (B) over the euvolemic region (D) is relatively flat and thus provides minimal information as to exactly where patient fluid volume resides within that region. Pressure-based diagnostic tools thus tend to only indicate measureable response after the patient's fluid state has entered into the hypovolemic region (O) or the hypervolemic region (R). In contrast, a diagnostic approach based on IVC diameter or area measurement across the respiratory and/or cardiac cycles (A₁ and A₂), which correlates directly to IVC volume and IVC CI (hereinafter "IVC Volume Metrics") provides relatively consistent information on patient fluid state across the full range of states.

Using IVC diameter or area measurement as an indicator of patient fluid volume as disclosed herein thus provides an opportunity for earlier response both as a hypovolemic warning and as an earlier hypervolemic warning. With respect to hypovolemia, when using pressure as a monitoring tool, a high pressure threshold can act as a potential sign of congestion, however when pressure is below a pressure threshold *(i.e.,* along the flat part of curve B), it gives no information about the fluid status as the patient approaches hypovolemia. With respect to hypervolemia, IVC diameter or area measurements potentially provide an earlier signal than pressure-based signals due to the fact that IVC diameter or area measurements change a relatively large amount without significant change in pressure. Hence, a threshold set on IVC diameter or area measurements can give an earlier indication of hypervolemia, in advance of a pressure-based signal.

Based on systems and methods disclosed herein, a patient healthcare provider can devise defined early warning zones for the hypovolemic region (O_{E}) and hypervolemic region (R_{E}). Just as the euvolemic region boundaries vary from patient to patient based on many physical and health related factors, such as age, sex, obesity and disease state. The early warning zones reside within the euvolemic range immediately adjacent the hypovolemic and hypervolemic regions such that the patient may still be considered to be within acceptable fluid balance parameters when in the early warning zones. However, the ability to define early warning zones as such based on IVC diameter or area measurements means that appropriate interventions may be initiated earlier, before the patient reaches higher levels of criticality, and thus also may be controlled more precisely and smoothly to minimize risk of shock from sudden interventions and/or overshoot of therapy targets. Table II below illustrates an example of possible fluid state regions for a hypothetical patient in accordance with the teachings of the present disclosure.

| **Table I - Example of Fluid State Regions for Hypothetical Patient** | | | | |
|---|---|---|---|---|
| Hypovolemic Region (O) | Hypovolemic Early Warning Zone (O_{E}) | Euvolemic Region | Hypervolemic Early Warning Zone (R_{E}) | Hypervolemic Range (R) |
| IVC **Ø** <14mm + IVCCI >75% | IVC Ø = 14-16mm + IVCCI = 60-75% | IVC Ø ~ 14-21mm + IVCCI ~ 50-75% | IVC Ø = 19-21mm + IVCCI = 50-60% | IVC Ø >21mm + IVCCI <50% |

FIG. 2 illustrates a hypothetical comparison of patient fluid volume over time in treatment for hypervolemia as between an IVC Volume Metrics-based approach according to the present disclosure (curve X) and a typical pressure-based approach (curve Y). Because of the greater information available in the euvolemic region, the IVC Volume Metrics-based approach permits more aggressive initial treatment with lower risk of overshoot into the hypovolemic region (low), resulting in bringing the patient into the euvolemic region (safe) more quickly as compared to a pressure-based system, which must modulate therapy more gradually. Curve Z illustrates the potential risk for a pressure-based system if treatment were initially applied in a manner similar to the IVC Volume Metrics-based system. Without the greater information and feedback available (as illustrated by FIG. 1, curves A₁ and A₂), by the time the treatment provider sees a response from the diagnostic tools, the aggressive treatment may have already pushed the patient into the hypovolemic region (low).

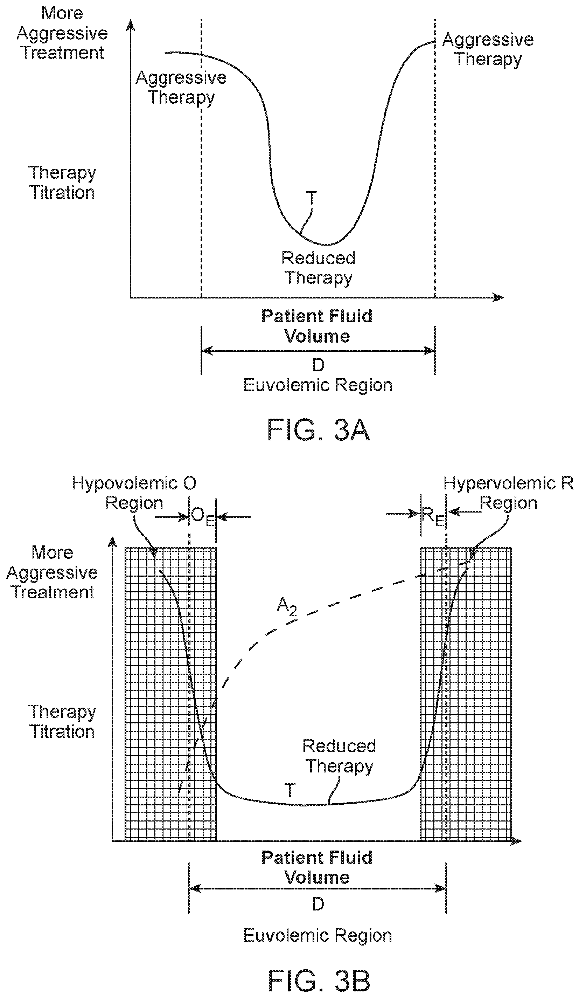

Use of IVC diameter or area measurements as described herein thus offers advantages in titrating patient therapies. FIGS. 3A and 3B illustrate embodiments of possible therapy titration schedules over the patient fluid state ranges based on the teachings of the present disclosure. Because IVC diameter/area changes more accurately reflect changes in patient fluid volume consistent with actual fluid state, IVC diameter or area measurements can be used to help titrate treatments more precisely and adjust the therapeutic intervention more subtly and incrementally, rather than just using a hard threshold as is now the clinical norm. Use of IVC diameter or area measurements also allows the flexibility of potentially titrating patients to a personalised volume, for example, keeping a patient with reduced cardiac ejection fraction (HFrEF) at a wetter point, while maintaining a patient with preserved cardiac ejection fraction (HFpEF) at a drier point.

FIG. 3A describes one possible treatment algorithm in this regard in which patient therapy is reduced when the patient's flood volume comfortably falls in the mid-range of the euvolemic region. In this treatment algorithm example, in which curve (T) represents a relative therapy level plotted against patient fluid volume, therapy is increased relatively rapidly once the patient's fluid volume moves from the mid-range of the euvolemic region as indicated by monitored changes in the IVC diameter or area. Such a treatment algorithm may be appropriate, for example, for a patient that is known to have a slow response to therapy in order to avoid having the patient move too far into the hypovolemic or hypervolemic regions before responding to the treatment. FIG. 3B describes another possible treatment algorithm based on the teachings of the present disclosure. In this example, relative therapy curve (T) is flatter across the majority of the euvolemic region (D) and only significantly increases once fluid volume, as determined based on sensed changes in IVC diameter or area, moves into one of the predefined early warning regions O_{E} or R_{E} that have been determined to be clinically appropriate for the specific patient being monitored. For illustration purposes, curve A₂ from FIG. 1 (representing change in IVC diameter/area vs. fluid volume) is superimposed over treatment curve (T) in FIG. 3B so that the relative relationship between IVC diameter/area change and treatment algorithm in this example may be better appreciated.

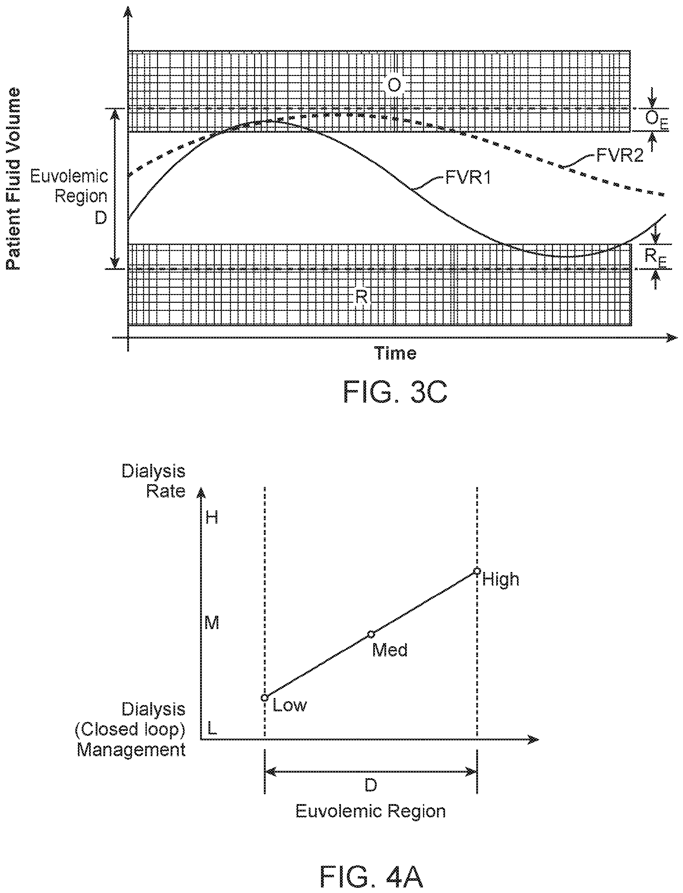

FIG. 3C schematically illustrates practical application of the relationships illustrated in FIG. 1 and potential advantages of treatment algorithms such as described in FIGS. 3A and 3B, based on sensed changes in IVC diameter or area as disclosed herein. In FIG. 3C, relative patient fluid volumes for hypothetical patients (whose therapy is titrated according to a treatment algorithm as described above) are plotted against relative time. Curves FVR1 and FVR2 thus represent two hypothetical examples of patient fluid volume response to therapy over time. In each case, applying a treatment algorithm such as described in the examples of FIGS. 3A or 3B, patient therapy can be titrated more accurately with respect to actual fluid state within the euvolemic region such that therapy may be applied at appropriate times earlier and more gradually to ensure that overall patient fluid volume stays within or as close as possible to the euvolemic region.

IVC diameter or area measurements also may be used in combination with other diagnostic signals to provide guidance on therapeutic intervention, e.g. diuretics versus vasodilators. When used with intervention, the IVC diameter or area measurement time dynamics response may be used to give information on the fluid status/distribution of the patient to guide therapy intervention. Response of IVC diameter or area measurements to a perturbation, e.g., physical activity, can cause sympathetic nerve response and fluid redistribution. Looking at changes in IVC diameter or area will thus provide information on fluid volume status. In other words, an act as simple as a leg raise may cause a fluid change/redistribution that could also provide information on fluid volume status that would not be visible with pressure-based systems. Thus, in certain embodiments, at-risk patients may have continuous or near-continuous monitoring of IVC diameter or area changes during physical activity.

Sensed changes in IVC diameter or area also may be combined with other parameters such as with BNP or pressure/edema signals to help guide therapy intervention or differentiate patient phenotype (HFrEF v HFpEF). Examples include detection of low collapsibility plus peripheral edema as an indication for diuretic therapy or detection of low collapsibility without peripheral edema as an indication for indicate vasodilator therapy. Combination of monitoring IVC diameter or area changes with implanted pressure-based monitors (in the IVC, right atrium, right ventricle, pulmonary artery, left atrium, or other vessel) also may permit determination of abdominal pressure and flow in the IVC. In addition, the IVC monitoring device of the invention may include additional sensors to measure non-dimensional parameters within the IVC such as blood flow rate and venous pressure. Further, measurement of the dimensions or non-dimensional parameters of other vessels, such as the superior vena cava, pulmonary artery, or heart chambers, may in some cases be advantageous to supplement IVC measurement. In such cases, dimensional measurement devices similar to the IVC monitoring device of the present invention may be configured for implantation in such other vessels. In such embodiments, the methods and systems of the invention may be adapted to receive such supplementary data from these sources and incorporate such data in the determination of fluid status, heart failure status, appropriate thresholds for communicating alerts or messages, or therapeutic treatment plans or recommendations.

Use of IVC diameter or area measurements also leads to the development of new systems such as closed-loop systems for therapy intervention as described herein. Examples include modification of a standard dialysis system filtration rate from a constant rate to a faster or variable rate using information that was previously unavailable to the clinician or patient. In one example, as illustrated in FIG. 4A, IVC diameter or area measurements may provide faster dialysis treatment in a closed-loop system, such as described below, by guiding higher filtration rates while the fluid load is high and inform reducing filtration rate as the fluid is reduced, ultimately resulting in a faster and safer treatment. Hypotensive events may occur in patients undergoing dialysis due to fluid removal occurring too rapidly. FIG. 4A plots patient fluid volume against rate of dialysis for a closed-loop system based on embodiments described herein, which may allow for more efficient dialysis, e.g., fast enough to remove fluid without the side effects of fast fluid removal. When ultrafiltration (UF) is constant, the degree of vascular refilling will differ from patient to patient, therefore using additional information provided by IVC diameter or area measurements may allow the UF rate to be more accurately individualized in a time dependent fashion over the course of the dialysis session for specific patients. IVC diameter or area measurement information may be combined with other diagnostic tools such as blood pressure monitoring to more accurately estimate fluid volume status as a basis for altering the rate of filtration.

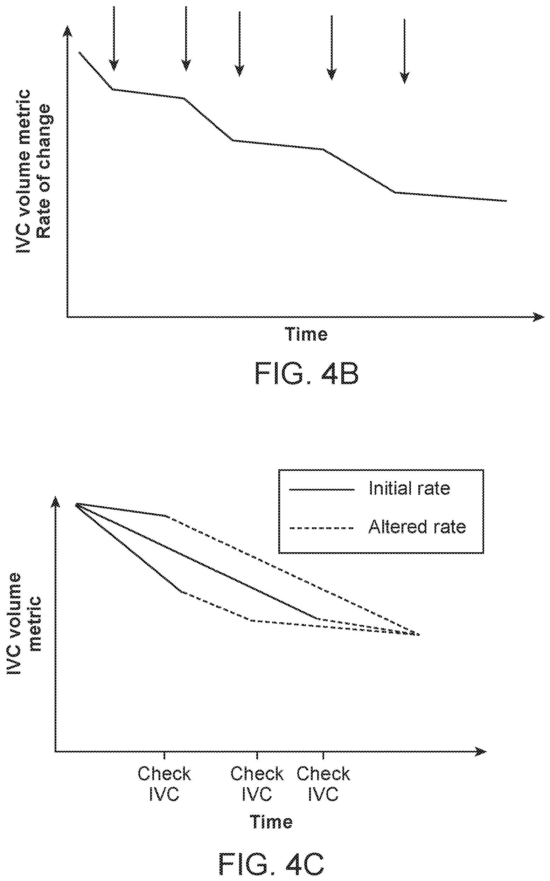

FIG. 4B illustrates another embodiment in which alteration of dialysis filtration rate may be based on periodic assessments of IVC diameter or area change, *e.g.,* a percent change in IVC volume metric per hour coinciding with a total desired volume that needs to be removed. (Each downward arrow in FIG. 4B indicates relative time of each assessment.) This is another alternative approach to control of UF rate, which allows increased accuracy and individualization of treatment for specific patients in a time dependent fashion over the course of a dialysis session. Another alternative dialysis control methodology is described in FIG. 4C in which the IVC volume metric rate of change, based on measured changes in IVC diameter or area, is plotted against time through a hypothetical dialysis session. Employing systems as described herein, time-based check points may be provided, at which time the measured IVC volume metric is checked against predefined patient specific targets. At each check point, UF rate may be altered as needed to direct the patient more efficiently and smoothly to the final fluid volume target. Compared to existing systems, which rely primarily on dry weight estimation based on inter-dialytic weight gain, employing methodologies as described in any of FIGS. 4A, 4B and 4C with systems disclosed herein provides for increased individualization of UF rate for specific patients over the course of the dialysis session.

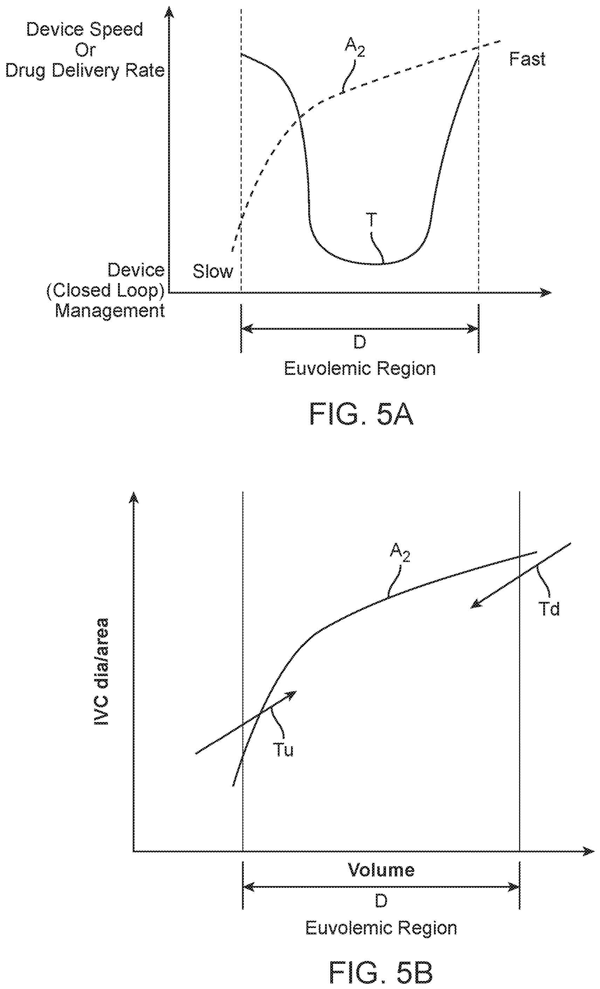

In another example, illustrated in FIG. 5A, an implanted drug pump or device may be provided, for example as a closed-loop dialysis management system. In such a system, at a hypervolemic end of the scale the device runs at high speed/delivers large load. As volume is reduced the device slows. This allows time for the interstitial fluid to return to the intravascular space. As the fluid load approaches hypovolemia the device speed / drug load rate could increase proportionally to avoid a hypovolemic state. Such control requires knowledge of incremental changes in fluid state across the euvolemic ranges, which is provided by methodologies and systems described herein. As a point of reference, curve A2 indicating relative IVC diameter or area measurement (from FIG. 1) is superimposed on the treatment curve (T) in FIG. 5A. In yet a further example, illustrated in FIG. 5B, a closed loop system according to embodiments described herein allows for volume control-based therapy delivery modulated based on measured changes in IVC diameter or area. In this example, at either end of the euvolemic region (D), therapy delivery, *e.g.,* drug delivery such as a diuretic or dialysis filtration, may be altered up (Tᵤ) or alerted down (T_{d}) in accordance with IVC diameter or area measurements.

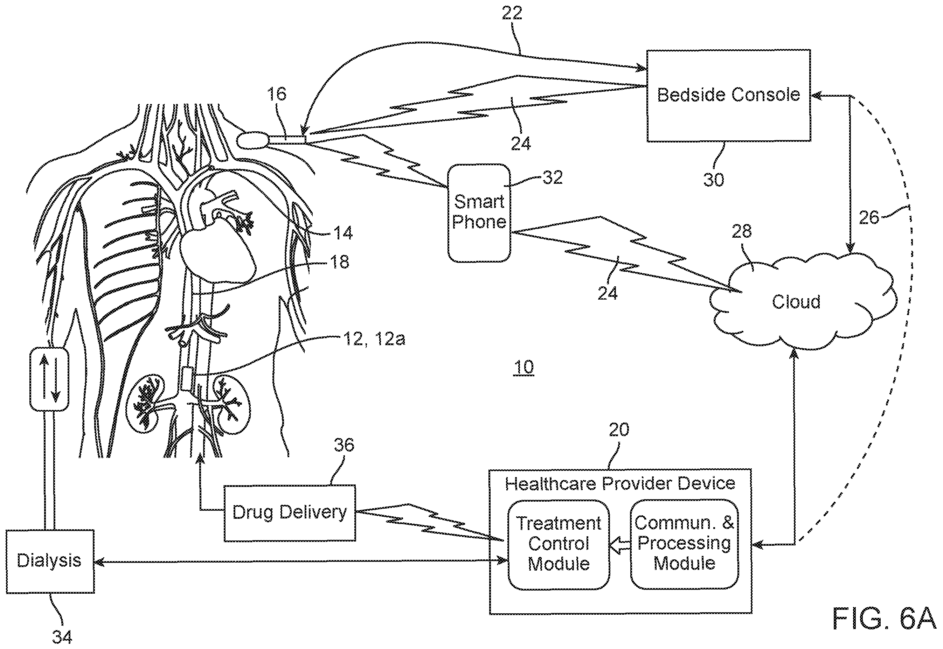

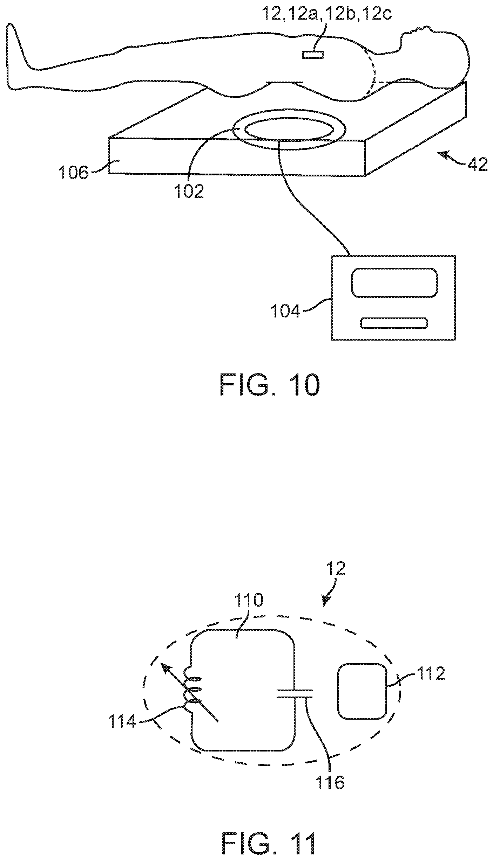

FIG. 6A schematically illustrates one exemplary system 10 including an IVC diameter/area measurement monitoring device 12 positioned at a monitoring location in the IVC. In the example illustrated, monitoring device 12 is an ultrasound-based device 12a anchored within the IVC and uses an ultrasound signal reflected off the opposite wall of the IVC to detect the distance by measuring the time-of-travel of this signal and thus provide a diameter measurement. Other examples of monitoring devices include resonant circuit-based devices in which characteristic inductance varies as the devices expand or contract with the IVC wall. Non-limiting examples of monitoring devices that may be used in systems according to the present disclosure are described below and shown in FIGS. 10, 11, and 12A-C. Further examples and details of suitable IVC diameter/area monitoring devices are disclosed in the aforementioned and incorporated PCT and provisional applications by the present Applicant.

Measurements of IVC diameter or area by monitoring device 12 may be made continuously over one or more respiratory cycles to determine the variation in IVC dimensions over this cycle. Further, these measurement periods may be taken continuously, at preselected periods and/or in response to a remotely provided prompt from a health care provider/patient. In this example, monitoring device 12 may communicate via an implanted antenna 14 positioned in the left brachiocephalic vein or other location close to an insertion point or location facilitating signal detection by an external antenna or detector 16. External antenna/detector 16 may be configured to be handheld by the patient or healthcare provider, or worn by or affixed to the patient in an anatomical location selected for optimal communication with the implanted antenna 14. Communication between the implanted antenna 14 and monitoring device 12 occurs via an intravascular lead 18, which extends through the venous vasculature to the monitoring device 12. This is just one example of a communications arrangement with a monitoring device such as device 12. In another example, wireless communication to receiver(s) outside the body may be effected directly by the monitoring device itself, without a separate, implanted antenna and connecting intravascular lead.

External antenna/detector 16 may be configured to communicate via wireless or wired connection with bedside console 30, smart phone 32, or other external communication/control device. Data collected by the monitoring device may be communicated ultimately to a healthcare provider device 20 via wired 22 and/or wireless 24 communications and/or directly through hard wired links such as telephone or local area networks 26 or through Internet or cloud based systems 28. Communications may be facilitated by a bedside console 30 in a home or clinical treatment location or, particularly in the case of implanted monitoring devices, through a mobile device 32, such as a smart phone. Healthcare provider device 20 may be configured with appropriate user interface, processing and communications modules for data input and handling, communications and processing, as well as treatment and control modules, which may include treatment algorithms as described herein for determining treatment protocols based on collected IVC diameter or area measurements, and systems for automated remote control of treatment devices based on determined treatment protocols as elsewhere described herein. Examples of such treatment devices include, but are not limited to, dialysis machine 34 and drug delivery devices 36. Examples of treatments include, when measured dimensions fall within the hypovolemic warning zone, administration of fluids or vaso-constricting drugs, and when measured dimensions fall within the hypervolemic warning zone, dialysis or administration of diuretics or vasodilating drugs.

IVC physical dimension data and/or fluid volume state information derived therefrom may also be communicated directly to the patient themselves, along with therapy advice based on this data and using pre-determined algorithms / implanted medical devices. Communications protocols throughout the system may include bidirectional communications to permit a healthcare provider (or other appropriately trained operator at another point in the system) to alter overall monitoring protocols executed at the monitoring device or, for example, to request additional queries by the monitoring device outside the current operational protocol.

Other embodiments include systems for patient self-directed therapy, for example with IVC volume metrics data utilized directly by the patient with or without clinician overview, e.g., for self-administration of drugs or other therapies. Such systems may also be implemented for home dialysis and/or peritoneal dialysis. Wireless communication between the IVC monitor and the patient's cell phone or computer would allow continuous or periodic transmission of IVC data and the use of software applications to provide alarms or reminders, graphically present trends, suggest patient actions, drug dosage options, or treatment system settings, and allow communication with physicians.

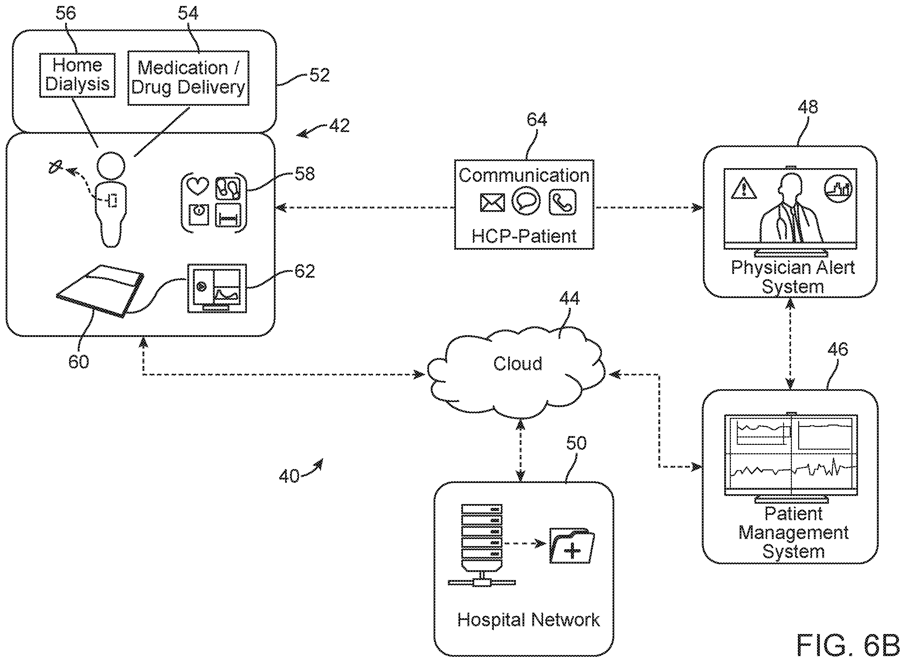

FIG. 6B schematically illustrates another exemplary system, which may, in one alternative, incorporate patient self-directed therapy. As shown in FIG. 6B, system 40 provides for communication between the patient home system 42, cloud storage 44, a patient management system 46, a physician alert system 48, and optionally a hospital network 50. Data transmission from the patient home system 42 to the cloud 44 for storage and access facilitates remote access for clinical and nursing teams. In patient self-directed therapy embodiments, patient's home may include home therapy devices 52, which may independently access cloud storage 44, and based on predetermined limits/treatment algorithms, indicate patient self-administration of medications or drug delivery 54 or home dialysis machines 56. In such a system a patient with wireless implant 12 may receive prompts from a cell phone or other device in the home at specific time intervals or in response to data 58 generated by other patient monitoring devices such as blood pressure, heart rate or respiration monitors that also communicate with the home device and may transmit data to cloud 44 for storage. System 40 may also include communication links (direct, networked or cloud-based) with such other monitoring devices to receive data 58 inputs used in setting warning zones and alert limits and assessing patient fluid state. Further inputs may be made by a user through a user interface, which may be, for example, configured as part of patient management system 46. User inputs may include additional patient-specific information such as patient age, sex, height, weight, activity level, or health history indicators.

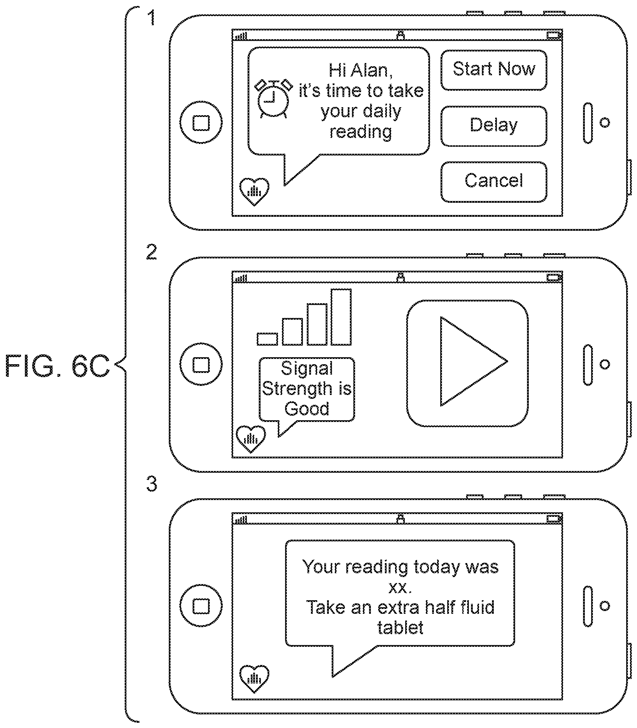

In response to a prompt from system 40 to take a reading, the patient would position him/herself with respect to antenna/detector 60 as appropriate to communicate with selected implant 12. Antenna/detector 60 may communicate locally with a control console 62 to receive and interpret signals from implant 12. FIG. 6C shows screen shots of a patient mobile device with examples of sequential prompts as may be provided on a home/mobile/cellular device.

Varying levels of response may be generated by the home system 42 depending on IVC measurements received from implant 12 and as may be interpreted in light of other patient data 58. Minimal responses may indicate to the patient that fluid status is within acceptable ranges and no action is required. Mid-level responses may include prompts for medication administration or changes in home drug delivery, or home dialysis. Examples of treatment protocols are explained further below. When home dialysis or drug delivery is prompted, it may be controlled directly in a closed-loop system as described above or may be controlled by the patient with prompts from the system. Patient data 58 and IVC measurements from implant 12 also may be communicated continuously or periodically by system 40 to cloud storage 44 and further communicated to a remote patient management system 46. Functionality for system 40 may be largely contained in home system 42 or in patient management system 46 or appropriately distributed across the network. Optionally, patient related data including sensor results and patient health and fluid states also may be communicated to or accessible by a hospital network 60. System 40 also may receive patient related data, including for example, medical records related to past therapies and medical history.

When a patient condition is recognized by system 40 as outside acceptable limits, an alert may be generated by physician alert system 48. Information supporting the alert condition may be communicated, for example, through patient management system 46 to physician alert system 48. Physician alert system 48 may reside at a healthcare provider office or may include a mobile link accessible by the health care provider remotely, and which permits communication 64 between the healthcare provider and the patient. Communication 64 between healthcare provider and patient may be network, Internet or telephone based and may include email, SMS (text) messaging or telephone/voice communication. Physician alert system 48 allows the healthcare provider to review logs of IVC measurements over time and make decisions regarding therapy titration, and in critical cases, hospital admissions, remote from the patient.

Exemplary system embodiments 10 and 40 are each illustrated, respectively, in FIGS. 6A and 6B with various system functions assigned to particular functional elements of the systems. For the sake of clarity of the disclosure, not all possible distributions of functions in functional elements across the system are described. As will be appreciated by persons of ordinary skill, other than the function of the sensor implant itself and, in some instances, an antenna communicating wirelessly with the sensor implant, all functions may be distributed among functional elements in any number of arrangements as best suited to a home or clinical application and the intended location of sensor reading function, *e.g.,* in a home or hospital setting. For example, all system functions (except sensor specific functions as mentioned) may be contained in a single functional unit in the form of a stand-alone patient management system. Alternatively, functions may be highly distributed among mobile devices networked with secure cloud computing solutions. For example, the sensor implant or, in cases where specific external antenna configuration is required, an antenna control module may communicate directly with a patient-owned smart phone to receive signals indicating IVC physical dimension measurements and, in turn, transmit those signals via WiFi or cell network to the cloud for distribution to further mobile devices in the possession of healthcare providers. Hand-held devices such as tablets or smart phones may communicate directly with controlled treatment delivery devices, or such devices may be controlled by a self-contained patient management system. Further, processing necessary for operation of the system also may be distributed or centralized as appropriate, or may be duplicated in multiple devices to provide safety and redundancy. As just one example, as shown in FIG. 6A, both bedside console 30 and smart phone 32 may be capable of performing identical functions and communicating with healthcare provider device 20 to report results of execution of the assigned functions. Thus, the specific arrangement of the functional elements (blocks) in the schematic presentations of the illustrative examples in FIG. 6A and 6B are not to be considered as limiting with respect to possible arrangements for distribution of disclosed functions across a network.

Various care algorithms may be developed based on systems 10 and 40. In one scenario, a first, home-care algorithm governs interactions in the home system including periodic IVC diameter/area measurements using implant 12 and dictates whether to maintain current therapies or to change therapies within the scope of home-care team capabilities. As long as IVC volume metrics stay within predefined limits, the first, home-care algorithm continues to govern monitoring and treatment. However, if monitored parameters, for example IVC volume metrics, exceed the predefined limits, then an alert is generated that engages a second, healthcare provider algorithm. Such an alert may be generated internally by home system 42, or may be generated in patient management system 46 (or physician alert system 48) based on monitored data communicated by home system 42 and received by the other systems either periodically or on a continuous basis. In one embodiment, an alert initially is received by a physician's assistant or heart failure nurse who can triage the situation through patient management system 46. At that level the assistant or nurse may elect to generate a message for communication 64 to the patient through the network related to modulation of therapy or other parameters such as level of physical activity. However, if triage indicates the alert to represent a more critical event, the physician may be alerted through physician alert system 48. Multiple layers of care and review based on measured IVC volume metrics are thus provided to efficiently manage patient fluid status and where possible avoid hospitalizations.

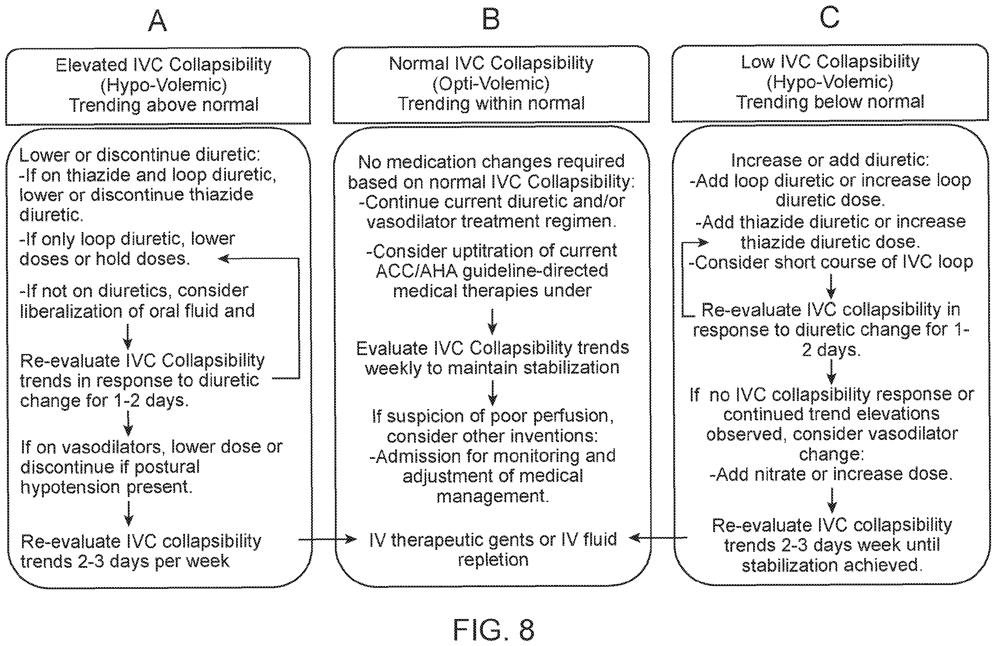

As mentioned above, IVC collapsibility or IVC CI are parameters that may be generated to facilitate diagnostic decisions based on IVC metrics. FIG. 7 illustrates one exemplary algorithm for determination of IVC collapsibility on which a treatment algorithm may be based. One example of such a treatment algorithm is illustrated in FIG. 8. Another example is described below in Table I. Plots A and B in FIG. 7 show two different IVC collapsibility conditions plotted as diameter versus time over several respiratory cycles (in this case based on ultrasound detection, but diameter/area detection of the IVC may be based on any other modalities described herein to achieve similar results).

Based on a calculated IVC collapsibility, a treatment algorithm such as shown in FIG. 8 may be employed. Based on several published research studies, an IVC Collapsibility Index (IVC CI) of 15% or less indicates significant fluid overload, which may imply an imminent risk of acute decompensation. An IVC CI of 20-30% might be considered normal, and an IVC CI of greater than 40% might indicate a hypovolemic state. These percentages may be adjusted for patients with certain conditions. For example, a patient with heart failure with reduced ejection fraction might preferably be maintained at a lower IVC CI *(i.e.,* with more circulating blood volume) to maximize cardiac output.

In developing any treatment algorithm a starting point is existing clinical guidelines, which a physician may then customize to an individual patient. Consistent with medically accepted best practices changes to treatment algorithms are made in conjunction with normal clinical exam and other data that treating physician has available. Embodiments described herein offer a new and powerful tool in this regard by making available regular IVC diameter or volume measurements without requiring a patient to be in a clinical setting and, potentially, providing continuous information on IVC volume metrics in near-real time.

With more and more accurate data on IVC volume metrics available to the healthcare provider based on systems described herein, more refined treatment algorithms may be devised. Such algorithms also may include a significant home-care component that was not previously possible. Table II below sets forth an alternative treatment algorithm in the form of IVC metrics to guide to patient volume status over the course of 4-5 respiratory cycles (IVC metrics employed in this algorithm include maximum and minimum diameters & IVCCI calculation (max-min)/max)x100).

| **TABLE II - Example of Treatment Algorithm** | | | | |
|---|---|---|---|---|
| **Measurement** | IVC **Ø** <14mm and IVCCI >75% | IVC **Ø** <21mm and IVCCI >50% | IVC **Ø** <21mm and IVCCI <50%, IVC **Ø** >21mm and IVCCI >50% | IVC **Ø** >21mm and IVCCI <50% , sniff or <20% quiet inspiration |
| **Characterize** | Low IVC Ø and high IVCCI (hypovolemic) | Normal IVC Ø and IVCCI (euvolemic) | Intermediate IVC Ø and IVCCI (intermediate) | Dilated IVC Ø, low IVCCI (hypervolemic) |
| **Trend** | Trending below normal | Trending within normal | Trending towards thresholds | Trending above normal |
| **Assessment** | Review diuretic dosing in line with the trend in IVC metric | No medication changes required based on normal metrics | Increase monitoring frequency | Consider increasing or adding diuretic |
| **Intervention or no intervention** | If on diuretic and other signs of hypovolemia are present omit half a diuretic dose until signal changes e.g. stop diuretic for 24-48 hrs | Continue current treatment regimen in line with current guideline driven standard of care, ensuring optimal dosing of one medicine | Consider up-titration of current medications in line with current guideline standard of care | Add or increase loop diuretic (e.g. 40mg furosemide or 1 mg bumetanide) |
| | | | | Add or increase thiazide or thiazide-like diuretic dose |
| | If not on diuretics, consider liberalization of oral fluid/salt | | | |
| | | | | Consider switching from furosemide to IV loop diuretic: initiate with 20-80mg |
| | If on vasodilators, lower dose or discontinue if postural hypotension present | | | |
| **Follow up** | Re-evaluate IVC trends in response to diuretic change for 2-3 days; adjust thresholds if necessary | Evaluate weekly to maintain stability | Evaluate 2x weekly to maintain stability; adjust thresholds if necessary | Re-evaluate IVC trends in response to diuretic change for 2-3 days |
| | | | | Measure renal function within 5-10 days of diuretic change: if creatinine increase by 20% or greater, consider reducing or discontinuing diuretic or reducing the vasoactive medication |
| **Additional actions** | n/a | n/a | n/a | If no IVC response or continued trend elevations observed, consider vasodilator change |

Treatment algorithms as described above may allow more precise titration and management of a patient's circulating blood volume than pulmonary artery pressure. As mentioned above, a patient might have a significant increase in circulating blood volume with only minor changes in pressure. Despite the normal pressure, this added volume may have deleterious short- and long-term effects on the patient's cardiac or renal function. By directly using IVC diameter or area measurements, the patient's fluid volume could be managed more closely, without a risk of inducing hypovolemia, which could also have deleterious effects.

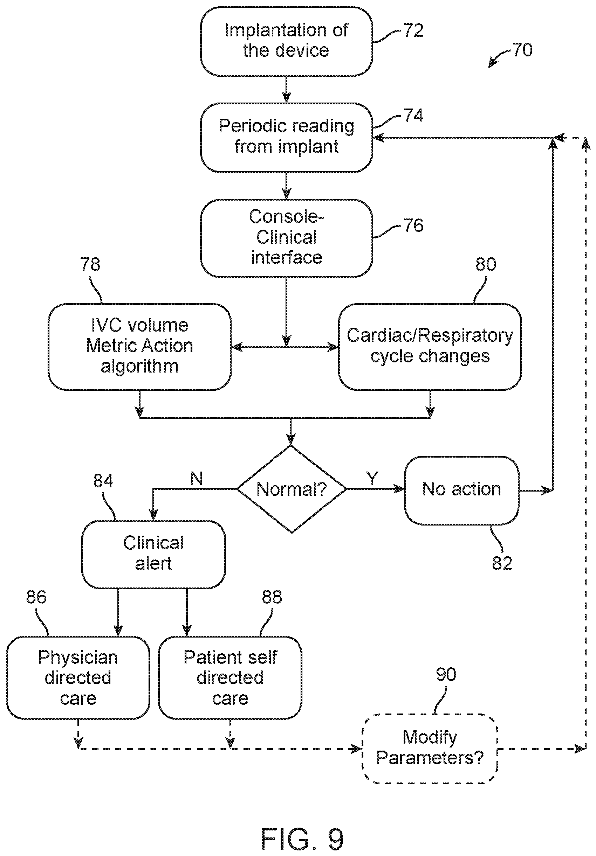

Utilizing embodiments described herein, it is possible to determine not only IVC metrics indicating blood volume status, but also respiration and heart rates. New clinical work flows also may be employed based on these multiple metrics to increase opportunities for improved patient outcomes. For example, as shown in FIG. 9, utilizing a system employing an implanted IVC diameter/area monitoring device, workflow 70 may include, for example, after device implantation 72, an initial detection algorithm that calls for periodic readings 74 of IVC diameter/area when the patient is at home. Such periodic readings may, for example, be taken weekly, daily or on other appropriate periods as determined by the healthcare provider based on patient parameters. In some embodiments the reading may be taken with the patient lying supine in bed and in proximity to a bedside console. Alternatively, the IVC diameter/area monitoring implant may include on-board memory, in which case it may also monitor IVC diameter or area measurements continuously or every few minutes and record the readings over the course of a day, and transmit once a day. Trend data for the selected period could be developed in this manner. Readings may be transmitted through the communications network as established to the clinical interface 76. Based on IVC metrics, *i.e.,* blood volume as determined in the clinical interface, the treatment algorithm determines necessary interventions if any. When conditions or trends are indicated within predetermined "normal" parameters for the specific patient, no action 82 is indicated and the system resets for the next periodic reading 74. However, if a condition or trend is indicated outside of the predetermined "normal" parameters, a clinical alert 84 may be generated and suggested interventions established by the applicable treatment algorithm employed. For example, in response to clinical alert 84, the healthcare provider directed care 86 or patient self-directed care 88 may be considered as suggested interventions and one or more effected consistent with the patient treatment plan. For patients already in a clinical setting, this may include instructions to other treatment devices connected to or working with the patient (for example, as shown in FIG. 6A with system 10). Other interventions or hospitalizations may be dictated for ambulatory patients or those otherwise outside a clinical setting when the alert is generated. Particularly for patients outside a clinical setting when an initial alert is generated, through bidirectional communication, the system allows the healthcare provider to instruct the monitoring device to generate one or more confirmatory monitoring signals before treatments are added or changed, or hospitalization required. After an intervention, the system may optionally reset for the next periodic reading 74. Depending on the nature or type of the initial clinical alert 84 and interventions 86, 88, patient parameters may be modified 90 by healthcare provider input or, optionally, in some cases, automatically by the system. Modifications may include, for example, changes in frequency of prompts for periodic readings 74 or changes in treatment algorithms that may be directed by the healthcare provider 86 or patient self-directed 88.

Further exemplary embodiments may include patient fluid management methods comprising steps such as measuring the diameter of the IVC in a patient, calculation of IVC collapsibility index and/or estimating patient blood volume based on IVC collapsibility, applying a treatment to the patient to effect a change in patient fluid level when determined fluid level is outside predetermined limits, continuously or substantially continuously monitoring IVC diameter or area measurements, such as change in IVC diameter, during said treatment and modulating said treatment in response to monitored change in the IVC diameter. With such methods, treatment modulation may be accomplished in near real-time as desired. The measurement and treatment may be directly linked and operate directly in a closed loop.

In one alternative, measurement of IVC diameter or area measurements may be performed by applying an electromagnetic signal from an external transmitter to a passive implant, and sensing the electromagnetic behavior of the passive implant in response to that signal using an external receiver. In another alternative, the measuring and monitoring may comprise positioning a monitoring device at a monitoring location within the IVC configured to detect a distance between opposed walls in the IVC or the diameter/area of the IVC at the monitoring location. Examples of suitable passive implants of this type are also disclosed in the aforementioned and incorporated PCT applications by the present Applicant.

Further alternative embodiments may involve monitoring IVC dimension variation over the respiratory and cardiac cycle, which may additionally include measurement/derivation of both breathing rate and heart rate on their own and/or in conjunction with different breathing maneuvers, or exercise. Longitudinal variation over days or weeks also may be a factor monitored. In another aspect, embodiments disclosed may include algorithms that incorporate other physiologic data, such as vascular pressures, heart rate, weight, posture, exercise status, etc. and also may use data from other implanted sensors, or other external devices.

In yet another alternative, the modulating may comprise use of multiple treatment algorithms including trend analysis and reference baselines with daily or near-daily titration of medications, diet, and other therapeutic actions. Diuretic delivery also may be added with algorithms generally applicable to patient populations, or custom algorithms based on specific patient status or physiology, for example, HFpEF vs HFrEF, renal functional status.

Other exemplary embodiments include fluid management systems comprising at least one monitoring device positioned in a patient IVC and configured to monitor IVC diameter or area measurements, such as changes in the IVC diameter, and output a signal representative of those changes. A healthcare provider device may be configured to communicate with the monitoring device in the patient IVC and determine patient treatment protocols based on the output signal and an executable treatment algorithm. Interventional devices are included providing patient treatment or therapies controlled by the healthcare provider device based on the determined treatment protocols.

A further alternative embodiment is a dialysis or ultrafiltration management method comprising continuously measuring the diameter of the IVC in a patient during dialysis or ultrafiltration, estimating patient blood volume based on measured IVC diameter, and adjusting the rate of fluid removal to continuously optimize the patient's circulating blood volume. The measurement of the diameter may track diameter variations over the respiratory and/or cardiac cycle. With such a method, a patient's circulating blood volume may be rapidly reduced to an optimal level at the beginning of the dialysis session, and then maintained at that level throughout the session as interstitial fluid migrates into the circulatory system. Further alternatives in such a method may be used to optimize the dialysis procedure so as to maximize safety, by preventing episodes of hypovolemia, effectiveness, by maximizing safe fluid removal from the interstitial space over a given time period and/or long-term patient health, by safely maintaining the patient at a lower total body fluid volume than could otherwise be maintained.

### Sensor Implant Examples

Examples of sensors 12 for use with systems and methods described herein are shown in FIGS. 10, 11, 12A, 12B and 12C. As mentioned previously, systems according to the present disclosure may generally comprise an implant 12 configured for placement in a patient's IVC. Such implants 12 may in some embodiments include control and communications modules, and one or more remote systems such as processing systems, user interface/displays, data storage, etc., communicating with the control and communications modules through one or more data links, preferably remote/wireless data links. FIG. 10 shows aspects of such systems, which in some embodiments may comprise all or part of home system 42 as shown in FIG. 6B. Such a system may include an antenna/detector module 102 to communicate with and, in some embodiments, power or actuate the implant. Antenna/detector module 102 is controlled by controller 104, which may comprise a bedside console as previously described. For patient comfort, as well as repeatability in positioning, antenna/detector module 102 may be place in a pad or bed 106.

One form of implant 12 may employ a variable inductance L-C circuit 110 for performing measuring or monitoring functions described herein, as shown in FIG. 11. Implant 12 may also include means 112 for securely anchoring the implant within the IVC. Using a variable inductor 114 and known capacitance 116, L-C circuit 110 produces a resonant frequency that varies as the inductance is varied. With the implant securely fixed at a known monitoring position in the IVC, changes in shape or dimension of the IVC cause a change in configuration of the variable inductor, which in turn cause changes in the resonant frequency of the circuit. These changes in the resonant frequency can be correlated to changes in the vessel shape or dimension by the implant control and communication system. Thus, not only should the implant be securely positioned at a monitoring position, but also, at least a variable coil/inductor portion 114 of the implant may have a predetermined compliance (resilience) selected and specifically configured to permit the inductor to move with changes in the vessel wall shape or dimension while maintaining its position with minimal distortion of the natural movement of the vessel wall. Thus, in some embodiments, the variable inductor is specifically configured to change shape and inductance in proportion to a change in the vessel shape or dimension.

Variable inductor 112 is configured to be remotely energized by an electric field delivered by one or more transmit coils within antenna/detector module102 positioned external to the patient. When energized, L-C circuit 110 produces a resonant frequency which is then detected by one or more receive coils of the antenna module. Because the resonant frequency is dependent upon the inductance of the variable inductor, changes in shape or dimension of the inductor caused by changes in shape or dimension of the vessel wall cause changes in the resonant frequency. The detected resonant frequency is then analyzed by the control and communication components of the system to determine the IVC diameter or area, or changes therein.

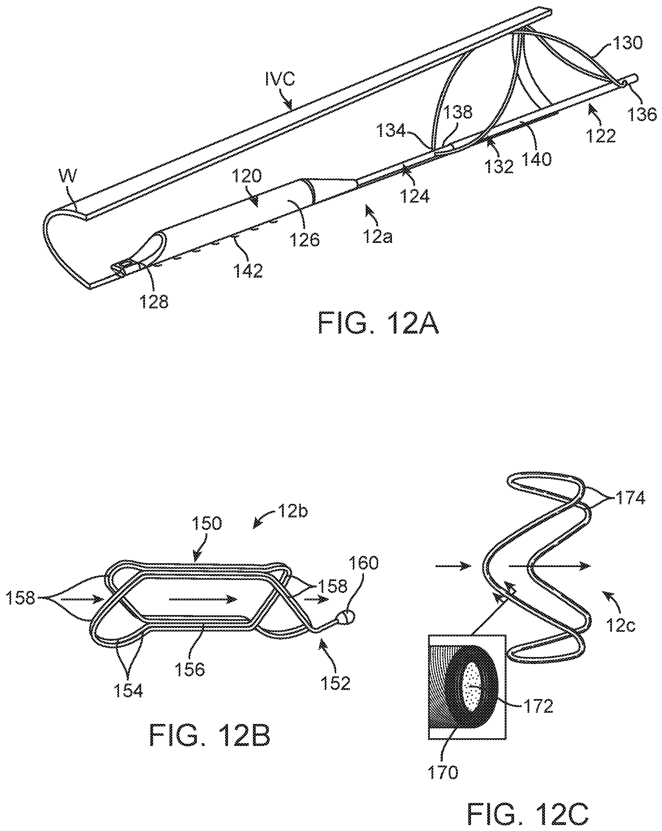

Turning to specific embodiments of implant 12, implant 12a, shown in FIG. 12, is an ultrasound-based device. As shown therein, 12a comprises three major components or assemblies, electronics capsule 120, anchor element 122 and anchor isolation structure 124 connecting the electronics capsule and anchor element. Electronics capsule 120 comprises a sealed housing 126 for containing control, power and other alternative functional modules as elsewhere described herein to provide a self-contained, sealed device. Capsule 120 also provides support for marker element 128, which in the case of implant 12a is a single ultrasound marker element positioned at the inferior end of the device. Such a marker element may utilize one or more ultrasound crystals to measure IVC diameter by emitting an ultrasound pulse, and then detecting the reflection of that pulse from the opposing wall of the IVC.

Electronics capsule 120 is connected to anchor element 122 at the superior end of the capsule. Anchor element 122 as depicted in this embodiment includes a single anchor wire 130 configured in a generally figure-eight or double helix shape. Alternatively, the same configuration can be provided with two or more wires. Anchor wire 130 is pinned to telescoping deployment member 132 at both its inferior end 134 and superior end 136. Telescoping deployment member 132 includes inner member 138, which is secured to electronics capsule 120, through anchor isolation structure 124 and outer member 140. Relative motion between inner member 138 and outer member 140 moves anchor wire 130 from a collapsed position to a deployed or anchoring position. Barbs 142 may be included to ensure fixation.

Various actuation mechanisms may be utilized for deploying and securing anchor element 122. In one alternative, anchor wire 130 is resilient, with shape-memory properties configured to provide a rest state in the deployed configuration. In this alternative, implant 12a may be delivered to the desired location in the IVC via a conventional guide catheter or other suitable sheath type delivery device. When position is confirmed as described below, implant 12a is ejected from the delivery catheter or sheath with anchor element 122 self-deploying upon ejection.

A further feature of implant 12a is spacing between the marker element position relative to the anchor element, provided by anchor isolation structure 124. In general, anchor element 122 is positioned sufficiently distant from the marker elements so as to not have an effect upon the IVC size or shape at or close to the location of measurement due to the anchoring force imparted to the IVC wall. Anchor isolation structure 124 facilitates the desired positioning, which may be distance approximately 1 to 4 times the IVC diameter from the measurement location.

FIGS. 12B and 12C illustrate further details of resonant circuit-based implants 12b and 12c, respectively. Implant 12b may comprise a "dog-bone"-like shape with a coil portion 150 and a capacitor portion 152. Implant 12b may comprise an electrically conductive wire or bundle of wires that is wound or otherwise formed into a single continuous coil comprising multiple turns or loops having an oval or rounded rectangular shape. It may be advantageous to use "Litz" wire, which has multiple independently insulated strands of wire, for the coil, since that may enhance the inductance of the implant. The coil is configured to be oriented such that the longer dimension of the rectangular loops extends longitudinally in a cranial-caudal direction within the IVC. The wire or group of wires may be wound multiple times in a continuous overlapping manner such that the rectangles each are defined by two or more parallel strands or bundles of wire about their periphery. The rectangles have central regions bounded by two or more longitudinal wires 154 forming spines 156 approximately defining a central plane running longitudinally in a cranial-caudal direction. This central region is configured to be disposed in a plane generally perpendicular to the anterior-posterior axis of the vessel, and remains relatively undeformed as the vessel collapses and expands in the anterior-posterior direction. The longitudinal elements may engage opposing walls of the vessel. At the caudal and cranial ends of the central regions of the rounded rectangles, the wire or wires form two lobes or a pair of coil ears 158 that flare outwardly away from each other and from the central plane of the implant in the anterior and posterior directions, as shown in FIG. 12B. Coil ears 158 are configured to engage opposing anterior and posterior walls of the vessel and to leave the central lumen of the vessel completely unobstructed for flow of blood as indicated by the arrows.

As the IVC changes shape, the longitudinal wires may move closer together or farther apart, and the coil ears may also move closer together or farther apart, thereby changing the inductance of the coil. The ears may be separated by about 1 cm to about 5 cm at the apex of the curved ends of the ears. An implant as adapted for an average IVC size may be about 2.5 cm to 10 cm long. It may be appreciated that as the IVC collapses in the anterior-posterior direction, the ears deform inwardly thereby changing the inductance of the coil. However, the central region of the coil remains relatively undeformed and maintains sufficient size that the inductance of the coil is high enough to produce a field sufficiently strong for external detection, as described more fully below. Capacitor portion 152 of implant 12b includes a capacitor element 160 to complete the RC circuit. Capacitor portion 152 can be located in a number of locations, such as distal to the ears, or along the spine.

FIG. 12C illustrates another alternative implant embodiment. The enlarged detail in the box of FIG. 12C represents a cross-sectional view taken as indicated. In this embodiment, implant 12c includes multiple parallel strands of wire 170 formed around a frame 172. With multiple strands of wires, the resonant circuit may be created with either the inclusion of a discrete capacitor, element or by the inherent inductance of the coils without the need for a separate capacitor as capacitance is provided between the wires 170 of the implant. Note that in the cross-sectional view of FIG. 12C, individual ends of the very fine wires are not distinctly visible due to their small size. The wires are wrapped around frame 172 in such a way to give the appearance of layers in the drawing. Exact capacitance required for the RC circuit can be achieved by tuning of the capacitance through either or a combination of discrete capacitor selection and material selection and configuration of the wires. In one alternative implant 12c, there may be relatively few wire strands, e.g. in the range of about 15 strands, with a number of loops in the range of about 20. In another alternative implant 12c, there may be relatively more wire strands, e.g., in the range of 300 forming a single loop.

Frame 172 may be formed from Nitinol, either as a shape set wire or laser cut shape. One advantage to a laser cut shape is that extra anchor features may cut along with the frame shape and collapse into the frame for delivery. When using a frame structure as shown in FIG. 12C, the frame should be non-continuous so as to not complete an electrical loop within the implant. As with the previous embodiment, coil wires may comprise fine, individually insulated wires wrapped to form a Litz wire. Factors determining inherent inductance include the number of strands and number of turns and balance of capacitance, Frequency, Q, and profile. One illustrative example of implant 12c may be configured as follows:
- 0.010" NiTi frame with 8 crowns (174 in FIG. 12C) - insulated with 0.013"x 0.00025" wall PET heat-shrink / parylene
- overall approximately 25-30mm diameter
- overall approximately 24mm long
- 25 turns, 25 strand, 46AWG gold Litz wire
- No discrete capacitor element - capacitance inherent in configuration of implant
- PET heat-shrink insulation (0.065" x 0.00025" wall) / parylene coated

Insertion of devices into the circulatory system of a human or other animal is well known in the art and so is not described in significant detail herein. Those of ordinary skill in the art will understand after reading this disclosure in its entirety that implants 12 can be delivered to a desired location in the IVC using, *e.g.,* a loading tool to load a sterile implant 12 into a sterile delivery system, which may be used to deliver the implant to the IVC via a femoral vein or other peripheral vascular access point, although other methods may be used.

### Computer - Software Implementation

It is to be noted that any one or more of the aspects and embodiments described herein, such as, for example, related to communications, monitoring, control or signal processing, may be conveniently implemented using one or more machines (e.g., one or more computing devices that are utilized as a user computing device for an electronic document, one or more server devices, such as a document server, etc.) programmed according to the teachings of the present specification. Appropriate software coding can readily be prepared by skilled programmers based on the teachings of the present disclosure, as will be apparent to those of ordinary skill in the software art. Aspects and implementations discussed above employing software and/or software modules may also include appropriate hardware for assisting in the implementation of the machine executable instructions of the software and/or software module.

Such software may be a computer program product that employs a machine-readable storage medium. A machine-readable storage medium may be any non-transitory medium that is capable of storing and/or encoding a sequence of instructions for execution by a machine *(e.g.,* a computing device) and that causes the machine to perform any one of the methodologies and/or embodiments described herein. Examples of a machine-readable storage medium include, but are not limited to, a magnetic disk, an optical disc *(e.g.,* CD, CD-R, DVD, DVD-R, etc.), a magnetooptical disk, a read-only memory "ROM" device, a random access memory "RAM" device, a magnetic card, an optical card, a solid-state memory device, an EPROM, an EEPROM, and any combinations thereof. A machine-readable medium, as used herein, is intended to include a single medium as well as a collection of physically separate media, such as, for example, a collection of compact discs or one or more hard disk drives in combination with a computer memory. As used herein, a machine-readable storage medium does not include transitory forms of signal transmission.

Such software may also include information (e.g., data) carried as a data signal on a data carrier, such as a carrier wave. For example, machine-executable information may be included as a data-carrying signal embodied in a data carrier in which the signal encodes a sequence of instruction, or portion thereof, for execution by a machine *(e.g.,* a computing device) and any related information *(e.g.,* data structures and data) that causes the machine to perform any one of the methodologies and/or embodiments described herein.

Examples of a computing device include, but are not limited to, an electronic book reading device, a computer workstation, a terminal computer, a server computer, a handheld device *(e.g.,* a tablet computer, a smartphone, smart watch, etc.), a web appliance, a network router, a network switch, a network bridge, any machine capable of executing a sequence of instructions that specify an action to be taken by that machine, and any combinations thereof. In one example, a computing device may include and/or be included in a kiosk.

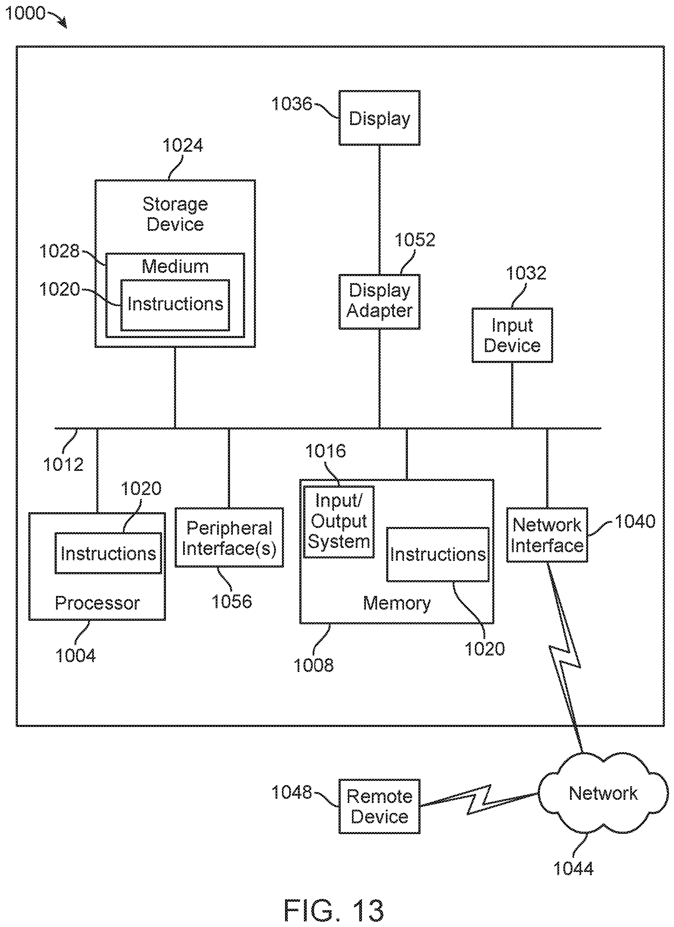

FIG. 13 shows a diagrammatic representation of one embodiment of a computing device in the exemplary form of an IVC diameter/area measuring implant control and communication system 1000 within which a set of instructions for causing an implant control and communication system, such as a waveform generator, an oscilloscope, an EFM circuit, or an implant, among other systems and devices disclosed herein, to perform any one or more of the aspects and/or methodologies of the present disclosure may be executed. It is also contemplated that multiple computing devices may be utilized to implement a specially configured set of instructions for causing one or more of the devices to perform any one or more of the aspects and/or methodologies of the present disclosure. Computer system 1000 includes a processor 1004 and a memory 1008 that communicate with each other, and with other components, via a bus 1012. Bus 1012 may include any of several types of bus structures including, but not limited to, a memory bus, a memory controller, a peripheral bus, a local bus, and any combinations thereof, using any of a variety of bus architectures.

Memory 1008 may include various components (e.g., machine-readable media) including, but not limited to, a random access memory component, a read only component, and any combinations thereof. In one example, a basic input/output system 1016 (BIOS), including basic routines that help to transfer information between elements within control and communication system 1000, such as during start-up, may be stored in memory 1008. Memory 1008 may also include *(e.g.,* stored on one or more machine-readable media) instructions *(e.g.,* software) 1020 embodying any one or more of the aspects and/or methodologies of the present disclosure. In another example, memory 1008 may further include any number of program modules including, but not limited to, an operating system, one or more application programs, other program modules, program data, and any combinations thereof.

Control and communication system 1000 may also include a storage device 1024. Examples of a storage device *(e.g.,* storage device 1024) include, but are not limited to, a hard disk drive, a magnetic disk drive, an optical disc drive in combination with an optical medium, a solid-state memory device, and any combinations thereof. Storage device 1024 may be connected to bus 1012 by an appropriate interface (not shown). Example interfaces include, but are not limited to, SCSI, advanced technology attachment (ATA), serial ATA, universal serial bus (USB), IEEE 1394 (FIREWIRE), and any combinations thereof. In one example, storage device 1024 (or one or more components thereof) may be removably interfaced with control and communication system 1000 *(e.g.,* via an external port connector (not shown)). Particularly, storage device 1024 and an associated machine-readable medium 1028 may provide nonvolatile and/or volatile storage of machine-readable instructions, data structures, program modules, and/or other data for control and communication system 1000. In one example, software 1020 may reside, completely or partially, within machine-readable medium 1028. In another example, software 1020 may reside, completely or partially, within processor 1004.

Control and communication system 1000 may also include an input device 1032. In one example, a user of control and communication system 1000 may enter commands and/or other information into control and communication system 1000 via input device 1032. Examples of an input device 1032 include, but are not limited to, an alpha-numeric input device *(e.g.,* a keyboard), a pointing device, a joystick, a gamepad, an audio input device *(e.g.,* a microphone, a voice response system, etc.), a cursor control device *(e.g.,* a mouse), a touchpad, an optical scanner, a video capture device *(e.g.,* a still camera, a video camera), a touchscreen, and any combinations thereof. Input device 1032 may be interfaced to bus 1012 via any of a variety of interfaces (not shown) including, but not limited to, a serial interface, a parallel interface, a game port, a USB interface, a FIREWIRE interface, a direct interface to bus 1012, and any combinations thereof. Input device 1032 may include a touch screen interface that may be a part of or separate from display 1036, discussed further below. Input device 1032 may be utilized as a user selection device for selecting one or more graphical representations in a graphical interface as described above.

A user may also input commands and/or other information to control and communication system 1000 via storage device 1024 *(e.g.,* a removable disk drive, a flash drive, etc.) and/or network interface device 1040. A network interface device, such as network interface device 1040, may be utilized for connecting control and communication system 1000 to one or more of a variety of networks, such as network 1044, and one or more remote devices 1048 connected thereto. Examples of a network interface device include, but are not limited to, a network interface card *(e.g.,* a mobile network interface card, a LAN card), a modem, and any combination thereof. Examples of a network include, but are not limited to, a wide area network *(e.g.,* the Internet, an enterprise network), a local area network *(e.g.,* a network associated with an office, a building, a campus or other relatively small geographic space), a telephone network, a data network associated with a telephone/voice provider *(e.g.,* a mobile communications provider data and/or voice network), a direct connection between two computing devices, and any combinations thereof. A network, such as network 1044, may employ a wired and/or a wireless mode of communication. In general, any network topology may be used. Information (e.g., data, software 1020, etc.) may be communicated to and/or from control and communication system 1000 via network interface device 1040.

control and communication system 1000 may further include a video display adapter 1052 for communicating a displayable image to a display device, such as display device 1036. Examples of a display device include, but are not limited to, a liquid crystal display (LCD), a cathode ray tube (CRT), a plasma display, a light emitting diode (LED) display, and any combinations thereof. Display adapter 1052 and display device 1036 may be utilized in combination with processor 1004 to provide graphical representations of aspects of the present disclosure. In addition to a display device, control and communication system 1000 may include one or more other peripheral output devices including, but not limited to, an audio speaker, a printer, and any combinations thereof. Such peripheral output devices may be connected to bus 1012 via a peripheral interface 1056. Examples of a peripheral interface include, but are not limited to, a serial port, a USB connection, a FIREWIRE connection, a parallel connection, and any combinations thereof.

As will be appreciated by persons of ordinary skill embodiments described herein may provide a number of beneficial effects and advantages as follows:
- As a fluid status indicator: Patients can be managed with greater confidence in euvolemia - *i.e.* with a greater margin of safety. The physician can take enough fluid off to restore some venous capacitance to act as a buffer against sudden fluid overload causing an acute decompensation - without taking so much fluid off as to cause kidney issues (IVC Volume Metric and collapsibility (IVC CI) are both key measures of patient's fluid status, and are more sensitive/responsive than pressure).
- As a decompensation risk indicator: As IVC diameter or area measurements (e.g. diameter) increase/ IVC collapsibility decreases (relative to an individual patient's baseline) it provides an earlier indicator of worsening fluid status, which in turn drives hemodynamic congestion, which drives clinical congestion (which may result in ADHF).
- As an aid to therapeutic decision making: Healthcare providers can use IVC Volume Metrics to indicate optimal diuresis point with an ability to provide longitudinal measures over a period of hours/ days/ weeks, helping the physician to factor in the impact of fluid redistribution (e.g., from the interstitial tissue into the intravascular space).
- As another aid to therapeutic decision making: IVC Volume Metrics assist healthcare providers in decision making as to whether to alter relative dosages of diuretics vis-à-vis vasodilators. For example, when a patient's cardiac pressure is increased, disclosed systems and methods facilitate important clinical decisions such as whether the cause is increased volume or increased vasoconstriction, whether to increase diuretics or vasodilators, whether to use IVC Volume Metrics to rule in/out increased volume as a primary cause of increased pressures, *i.e.,* if increased volume is confirmed then diuretics may be indicated, if not then vasodilators may be indicated
- Algorithm based on inputs and output as disclosed also:
   o Assesses IVC metric
   o Compares daily result and trend to guideline based limits (and over time patient specific limits)
   o Determines if medication modification is required
   o Sends signal / message to patient and requests confirmation of medication alteration
   o By exception (based on multiple times exceeding limits / trends / other trigger)
   o Sends notification to managing physician
   o Physician can then use system to send message to patient to modify medication and confirm change
   o System may provide alarm to remind patient to take medication / take reading
   o All this info is stored in the cloud server

The foregoing has been a detailed description of illustrative embodiments of the disclosure. It is noted that conjunctive language such as is used herein in phrases like "at least one of X, Y and Z" and "one or more of X, Y, and Z," unless specifically stated or indicated otherwise, shall be taken to mean that each item in the conjunctive list can be present in any number exclusive of every other item in the list or in any number in combination with any or all other item(s) in the conjunctive list, each of which may also be present in any number. Applying this general rule, the conjunctive phrases in the foregoing examples in which the conjunctive list consists of X, Y, and Z shall each encompass: one or more of X; one or more of Y; one or more of Z; one or more of X and one or more of Y; one or more of Y and one or more of Z; one or more of X and one or more of Z; and one or more of X, one or more of Y and one or more of Z.

The invention is defined by the appended claims.

## Claims

1. A system for managing patient body fluid volume, comprising:
a sensing device (12) for directly measuring a physical dimension of the patient's Inferior Vena Cava (IVC) when implanted within the IVC;
a sensing control module (30, 32) for wirelessly communicating with the implanted sensing device to monitor changes in the measured IVC dimension throughout a euvolemic region defined for said patient; and
a patient management system (46) for generating an alert signal when IVC metrics based at least in part on the measured IVC physical dimension indicate the patient's fluid volume state meets predetermined clinical limits for the defined euvolemic region; wherein said predetermined clinical limits comprise at least an indication of the patient fluid state trending towards hypovolemia, and an indication of the patient fluid state trending towards hypervolemia; and wherein each of said predetermined limits falls within the euvolemic range defined for the patient; **characterised in that** said predetermined clinical limits comprise:
a hypovolemic warning zone (O_{E}) for said patient encompassing a portion of the defined euvolemic region at a lower end of the euvolemic region adjacent a hypovolemic region; and
a hypervolemic warning zone (R_{E}) for said patient encompassing a portion of the defined euvolemic region at an upper end of the euvolemic region adjacent a hypervolemic region, wherein said euvolemic range, hypovolemic range and hypervolemic range are correlated to IVC physical dimension measurements for said patient; and wherein the patient management system is configured to generate an alert signal when said IVC metrics indicate the patient fluid state falls within either of the hypovolemic warning zone or the hypervolemic warning zone of the defined euvolemic region before the patient fluid state reaches one of the hypovolemic region or hypervolemic region, respectively.

2. The system of claim 1, wherein said physical dimension of the patient's IVC is at least one of IVC diameter or IVC area.

3. The system of claim 1, further adapted to receive data inputs and use the data inputs in setting warning zones and alert limits.

4. The system of any preceding claim, wherein said IVC metrics are determined based on a combination of measured and calculated data including at least a measured IVC physical dimension and calculated collapsibility index.

5. The system of claim 4, wherein the patient management system is further configured to determine patient fluid volume state based on said combination of measured and calculated data; and
generate a patient level alert when patient fluid volume status is determined to fall within a first range, and to generate a clinical level alert when patient fluid volume status is determined to fall within a second range.

6. The system of claim 5, further comprising at least one of:
a patient device configured to receive said patient level alert; and
a healthcare provider device configured to receive said clinical level alert.

7. The system of any preceding claim, further comprising a user interface configured for user input of patient specific information and a communication link with diagnostic devices monitoring patient condition.

8. The system of any preceding claim, wherein:
said wireless sensing device comprises a resilient coil forming a resonant circuit, said resilient coil configured to engage the wall of the IVC and deform therewith to provide a variable characteristic frequency correlated to an IVC physical dimension in response to activation of the resonant circuit; and
said system further comprises an antenna configured to activate the resonant circuit and receive the signal representative of the measured IVC physical dimension in response to said activation.

9. The system of any preceding claim further comprising means for communicating IVC measurements from the wireless sensing device implant to cloud storage for determining patient treatment protocols based on the measurements and an executable treatment algorithm.

10. The system any preceding claim, wherein said alert signal includes a prompt to initiate a patient self-directed treatment or treatment change.

## Patentansprüche

1. Ein System zum Verwalten des Körperflüssigkeitsvolumens eines Patienten, das Folgendes aufweist:
eine Erfassungseinrichtung (12) zum direkten Messen einer physikalischen Abmessung der Vena cava inferior (IVC) des Patienten, wenn sie innerhalb der IVC implantiert ist;
ein Erfassungssteuerungsmodul (30, 32) zum drahtlosen Kommunizieren mit der implantierten Erfassungseinrichtung, um Änderungen der gemessenen IVC-Abmessung innerhalb einer euvolämischen Region zu überwachen, die für den besagten Patienten definiert ist; und
ein Patientenmanagementsystem (46) zum Erzeugen eines Alarmsignals, wenn IVC-Metriken, die wenigstens teilweise auf der gemessenen physikalischen IVC-Abmessung basieren, anzeigen, dass der Flüssigkeitsvolumenstatus des Patienten vorgegebene klinische Grenzwerte für die definierte euvolämische Region erfüllt;
wobei die besagten vorgegebenen klinischen Grenzwerte wenigstens eine Angabe, dass der Flüssigkeitsstatus des Patienten zur Hypovolämie tendiert, und eine Angabe, dass der Flüssigkeitsstatus des Patienten zur Hypervolämie tendiert, aufweisen; und
wobei jeder der besagten vorgegebenen Grenzwerte innerhalb des für den Patienten definierten euvolämischen Bereichs liegt;
**dadurch gekennzeichnet, dass** die besagten vorgegebenen klinischen Grenzwerte Folgendes aufweisen:
eine hypovolämische Warnzone (O_{E}) für den besagten Patienten, die einen Teil der definierten euvolämischen Region an einem unteren Ende der euvolämischen Region, das an eine hypovolämische Region angrenzt, umfasst; und
eine hypervolämische Warnzone (R_{E}) für den besagten Patienten, die einen Teil der definierten euvolämischen Region an einem oberen Ende der euvolämischen Region, das an eine hypervolämische Region angrenzt, umfasst,
wobei der besagte euvolämische Bereich, hypovolämische Bereich und hypervolämische Bereich mit Messungen der physikalischen IVC-Abmessung für den besagten Patienten korreliert sind; und
wobei das Patientenmanagementsystem eingerichtet ist zum Erzeugen eines Alarmsignals, wenn die besagten IVC-Metriken anzeigen, dass der Flüssigkeitsstatus des Patienten in die hypovolämische Warnzone oder die hypervolämische Warnzone der definierten euvolämischen Region fällt, bevor der Flüssigkeitsstatus des Patienten die hypovolämische Region oder die hypervolämische Region jeweils erreicht.

2. Das System nach Anspruch 1, wobei die besagte physikalische Abmessung der IVC des Patienten der IVC-Durchmesser und/oder die IVC-Querschnittsfläche ist.

3. Das System nach Anspruch 1, ferner angepasst zum Empfangen von Dateneingaben und zum Verwenden der Dateneingaben beim Einstellen von Warnzonen und Alarmgrenzwerten.

4. Das System nach einem der vorhergehenden Ansprüche, wobei die besagten IVC-Metriken basierend auf einer Kombination aus gemessenen und berechneten Daten bestimmt werden, die wenigstens eine gemessene physikalische IVC-Abmessung und einen berechneten Kollapsibilitätsindex aufweisen.

5. Das System nach Anspruch 4, wobei das Patientenmanagementsystem ferner eingerichtet ist zum
Bestimmen des Flüssigkeitsvolumenstatus des Patienten basierend auf der besagten Kombination aus gemessenen und berechneten Daten; und
Erzeugen eines Alarms auf Patientenebene, wenn bestimmt wird, dass der Flüssigkeitsvolumenstatus des Patienten in einen ersten Bereich fällt, und zum Erzeugen eines klinischen Alarms, wenn bestimmt wird, dass der Flüssigkeitsvolumenstatus des Patienten in einen zweiten Bereich fällt.

6. Das System nach Anspruch 5, ferner wenigstens eines der Folgenden aufweisend:
eine Patienteneinrichtung, eingerichtet zum Empfangen des besagten Alarms auf Patientenebene; und
eine Einrichtung für Gesundheitsdienstleister, eingerichtet zum Empfangen des besagten klinischen Alarms.

7. Das System nach einem der vorhergehenden Ansprüche, ferner eine Benutzerschnittstelle aufweisend, eingerichtet zur Benutzereingabe patientenspezifischer Informationen und zur Kommunikationsverbindung mit Diagnoseeinrichtungen, die den Patientenzustand überwachen.

8. Das System nach einem der vorhergehenden Ansprüche, wobei:
die besagte drahtlose Erfassungseinrichtung eine elastische Spule aufweist, die eine Resonanzschaltung ausbildet, wobei die besagte elastische Spule eingerichtet ist zum Anliegen an die Wand der IVC und zum Verformen zusammen mit dieser, um eine variable charakteristische Frequenz bereitzustellen, die mit einer physikalischen IVC-Abmessung in Reaktion auf eine Aktivierung der Resonanzschaltung korreliert ist; und
das besagte System ferner eine Antenne aufweist, eingerichtet zum Aktivieren der Resonanzschaltung und zum Empfangen des Signals, das für die gemessene physikalische IVC-Abmessung repräsentativ ist, und zwar in Reaktion auf die besagte Aktivierung.

9. Das System nach einem der vorhergehenden Ansprüche, ferner aufweisend Mittel zum Kommunizieren von IVC-Messungen von der implantierten drahtlosen Erfassungseinrichtung zum Cloudspeicher, zum Bestimmen von Patientenbehandlungsprotokollen basierend auf den Messungen und einem ausführbaren Behandlungsalgorithmus.

10. Das System nach einem der vorhergehenden Ansprüche, wobei das besagte Alarmsignal eine Aufforderung zum Einleiten einer patientengesteuerten Behandlung oder Behandlungsänderung enthält.

## Revendications

1. Système de gestion du volume de fluide corporel du patient, comprenant :
un dispositif de détection (12) pour mesurer directement une dimension physique de la veine cave inférieure (VCI) du patient lorsqu'il est implanté dans la VCI ;
un module de contrôle de détection (30, 32)
pour assurer une communication sans fil avec le dispositif de détection implanté afin de surveiller les changements dans la dimension de la VCI mesurée tout au long d'une région euvolémique définie pour ledit patient ; et
un système de gestion du patient (46) pour générer un signal d'alerte lorsque les métriques de la VCI basés au moins en partie sur la dimension physique de la VCI mesurée indiquent que l'état du volume de fluide du patient atteint les limites cliniques prédéterminées pour la région euvolémique définie ;
dans lequel lesdites limites cliniques prédéterminées comprennent au moins une indication de l'état du fluide du patient tendant vers l'hypovolémie, et une indication de l'état du fluide du patient tendant vers l'hypervolémie ; et dans lequel chacune desdites limites prédéterminées se situe dans la plage euvolémique définie pour le patient ;
**caractérisé en ce que** lesdites limites cliniques prédéterminées comprennent :
une zone d'alerte hypovolémique (O_E) pour ledit patient englobant une portion de la région euvolémique définie à une extrémité inférieure de la région euvolémique adjacente à une région hypovolémique ; et
une zone d'alerte hypervolémique (R_E) pour ledit patient englobant une portion de la région euvolémique définie à une extrémité supérieure de la région euvolémique adjacente à une région hypervolémique, dans lequel ladite plage euvolémique, plage hypovolémique et plage hypervolémique sont corrélées aux mesures de la dimension physique de la VCI pour ledit patient ; et
dans lequel le système de gestion du patient est configuré pour générer un signal d'alerte lorsque lesdits paramètres de la VCI indiquent que l'état du fluide du patient se situe dans l'une quelconque de la zone d'alerte hypovolémique ou de la zone d'alerte hypervolémique de la région euvolémique définie avant que l'état du fluide du patient n'atteigne l'une quelconque de la région hypovolémique ou de la région hypervolémique, respectivement.

2. Système selon la revendication 1, dans lequel ladite dimension physique de la VCI du patient est au moins l'un quelconque du diamètre de la VCI ou de la surface de la VCI.

3. Système selon la revendication 1, en outre adapté pour recevoir des entrées de données et utiliser lesdites entrées de données pour définir des zones d'alerte et des seuils d'alerte.

4. Système selon l'une quelconque des revendications précédentes, dans lequel lesdits métriques de la VCI sont déterminés sur la base d'une combinaison de données mesurées et de données calculées comprenant au moins une dimension physique de la VCI mesurée et un indice de collapsibilité calculé.

5. Système selon la revendication 4, dans lequel le système de gestion du patient est en outre configuré pour déterminer l'état du volume de fluide du patient sur la base de ladite combinaison de données mesurées et de données calculées ; et
générer une alerte au niveau du patient lorsque le statut du volume de fluide du patient est déterminé comme se situant dans une première plage, et générer une alerte au niveau clinique lorsque le statut du volume de fluide du patient est déterminé comme se situant dans une seconde plage.

6. Système selon la revendication 5, comprenant en outre au moins l'un quelconque de :
un dispositif du patient configuré pour recevoir ladite alerte au niveau du patient ; et
un dispositif du prestataire de soins configuré pour recevoir ladite alerte au niveau clinique.

7. Système selon l'une quelconque des revendications précédentes, comprenant en outre une interface utilisateur configurée pour la saisie utilisateur d'informations spécifiques au patient et un lien de communication avec des dispositifs de diagnostic surveillant l'état du patient.

8. Système selon l'une quelconque des revendications précédentes, lequel :
le dispositif de détection sans fil comprend une bobine résiliente formant un circuit résonant, ladite bobine résiliente étant configurée pour engager la paroi de la VCI et se déformer avec celle-ci afin de fournir une fréquence caractéristique variable corrélée à une dimension physique de la VCI en réponse à l'activation du circuit résonant ; et
ledit système comprend en outre une antenne configurée pour activer le circuit résonant et recevoir le signal représentatif de la dimension physique de la VCI mesurée en réponse à ladite activation.

9. Système selon l'une quelconque des revendications précédentes, comprenant en outre des moyens pour communiquer les mesures de la VCI depuis le dispositif de détection sans fil implanté vers le stockage en nuage afin de déterminer des protocoles de traitement du patient sur la base des mesures et d'un algorithme de traitement exécutable.

10. Système selon l'une quelconque des revendications précédentes, dans lequel ledit signal d'alerte comprend une invite pour initier un traitement autodirigé par le patient ou une modification du traitement.
